# EUROPEAN PATENT SPECIFICATION

(11) **EP 4 337 007 B1**
(45) Date of publication and mention of the grant of the patent: **15.07.2026**
(21) Application number: 22808100.6
(22) Date of filing: 06.05.2022
(51) Int. Cl.: C12N 5/00, B01L 3/00, G06K 17/00, A01N 1/10, A01N 1/14, A01N 1/147, G06K 7/10, A01N 1/145

(54) **TRANSFER STATION, INTERROGATION STATION, AND RELATED METHODS TO FACILITATE TRANSFER OF BIOLOGICAL SPECIMENS STORED AT CRYOGENIC CONDITIONS**
TRANSFERSTATION, ABFRAGESTATION UND ZUGEHÖRIGE VERFAHREN ZUR ERLEICHTERUNG DER ÜBERTRAGUNG VON UNTER KRYOGENEN BEDINGUNGEN GELAGERTEN BIOLOGISCHEN PROBEN
STATION DE TRANSFERT, STATION D'INTERROGATION ET PROCÉDÉS ASSOCIÉS POUR FACILITER LE TRANSFERT D'ÉCHANTILLONS BIOLOGIQUES STOCKÉS DANS DES CONDITIONS CRYOGÉNIQUES

(30) Priority: 10.05.2021 US 202163186593 P
(43) Date of publication of application: 20.03.2024
(73) Proprietor: TMRW Life Sciences, Inc., New York NY 10013 (US)
(72) Inventor: BIXON, Brian, New York, NY 10013 (US); HULTGREN, Alexander, New York, NY 10013 (US); CRAVEN, James, New York, NY 10013 (US)
(74) Representative: Meissner Bolte Düsseldorf Patentanwälte Rechtsanwälte Partnerschaft mbB
(86) International application number: PCT/US2022/028185
(87) International publication number: WO 2022/240699

(56) References cited:
- EP-A2- 0 411 224
- WO-A1-2021/086983
- WO-A1-2022/066943
- CN-A- 106 102 460
- CN-A- 107 624 751
- US-A1- 2010 302 040
- US-A1- 2020 107 541
- US-A1- 2020 229 429
- US-A1- 2021 121 876
- ANONYMOUS: "BioStoreTM III Automated LN2 Sample Storage Solutions", 17 March 2020 (2020-03-17), Internet, pages 1 - 6, XP093203686, Retrieved from the Internet <URL:https://www.conferenceharvester.com/uploads/harvester/VirtualBooths/12905/BPFLSJUN-PDF-2-443738.pdf>

## Description

### Technical Field

The present disclosure generally relates to a transfer station, interrogation station, and related methods to transfer biological specimens (*e.g.,* eggs, sperm, embryos, other biological tissue) between a cryogenic freezer (*e.g.,* cryogenic storage tank or liquid nitrogen bath), which is typically fixed or stationary, and a portable thermally insulated carrier or cassette, and to facilitate identification of stored biological specimens and evidence chain-of-custody during handling.

### BACKGROUND

### Description of the Related Art

Long-term preservation of cells and tissues through cryopreservation has broad impacts in multiple fields including tissue engineering, fertility and reproductive medicine, regenerative medicine, stem cells, blood banking, animal strain preservation, clinical sample storage, transplantation medicine, and in vitro drug testing. This can include the process of vitrification in which a biological specimen or sample (*e.g.,* an oocyte, an embryo, a biopsy) contained in or on a storage device (*e.g.,* a cryopreservation straw, cryopreservation tube, stick or spatula) is rapidly cooled by placing the biological specimen and the storage device in a substance, such as liquid nitrogen.

This results in a glass-like solidification or glassy state of the biological specimen *(e.g.,* a glass structure at the molecular level), which maintains the absence of intracellular and extracellular ice (*e.g.,* reducing cell damage and/or death) and, upon thawing, improves post-thaw cell viability. To ensure viability, the vitrified biological specimens must then be continuously stored in a liquid nitrogen dewar or other container containing the liquid nitrogen, which is at a temperature of negative 190 degrees Celsius.

Each harvested biological specimen is loaded on a rigid specimen holder (*e.g.,* embryo straw, stick or spatula). In the case of a tubular specimen holder, the tube may be closed (*e.g.,* plugged) at one end and open at the other end. The cryopreservation storage devices (*e.g.,* specimen holders) containing or holding the biological specimen are cooled as quickly as possible by plunging the cryopreservation storage device with the biological specimen into a liquid nitrogen bath in a cryogenic freezer at a temperature of approximately negative 190 degrees Celsius, for example to achieve vitrification. More particularly, multiple cryopreservation storage devices are placed in a goblet for placement in the liquid nitrogen storage tank or freezer. The goblet attaches to the liquid nitrogen storage tank such that the multiple cryopreservation storage devices are suspended in the liquid nitrogen.

Labels that are manually written-on using a suitable marker pen or printed using a custom printer are attached to the straw and/or the goblet. Such labels can include identification information corresponding to the individual that the embryo was harvested from and other suitable information (*e.g.,* a cryopreservation storage device number, a practitioner number, etc.).

Access to the biological specimens is required during normal operation. For example, a particular biological specimen or specimens may be required to perform a procedure (*e.g.,* implantation of a fertilized egg). Retrieval of cryopreservation storage devices and associated biological specimens from the cryogenic refrigerator or cryogenic tank in which the biological specimens are stored exposes the retrieved biological specimens to non-cryogenic conditions (*e.g.,* temperatures above negative 190° C), and depending on a duration of the exposure places the biological specimens at risk.

Due to the way biological specimens are stored (*e.g.,* cryopreservation storage devices arrayed in cassettes, cassettes arrayed in stacks), retrieval of one or more desired biological specimens often requires retrieval of additional biological specimens that are not needed at that time, exposing such to risk. Additionally, transport of biological specimens from a cryogenic refrigerator to a site (i.e., worksite or work station) of an intended use (*e.g.,* fertilization, implantation) exposes the biological specimens to risk.

Thus, there are a number of concerns in how biological specimens are being stored, identified, managed, inventoried, retrieved, etc. With regard to storage and management of these biological specimens, facilities employ personnel that are required to maintain the liquid nitrogen storage tanks (*e.g.,* by refilling them with liquid nitrogen when needed) and manage the inventory of stored biological specimens (*e.g.,* by performing periodic accountings). There is, however, little recordkeeping with regard to the proper storage of these biological specimens. For example, subsequent identification or otherwise handling of the vitrified biological specimen or sample can involve removal of the specimen from temperature-controlled storage and exposure of the sample to ambient temperature, thus potentially risking the viability of the sample.

WO 2022 066943 A1 describes a system and method for transferring specimen containers, such as vials with caps, between storage cassettes and carrier cassettes. The storage cassettes are intended for cryogenic refrigeration, while the carrier cassettes are designed for temporary use in a portable carrier. The workstation includes a well with removable buckets sized to hold the respective cassettes. One or more arrays of antennas are positioned beneath the well to interrogate wireless transponders attached to the specimen containers. The invention also includes improved designs for both storage and carrier cassettes.

WO 2021 086983 A1 describes a system and method for transferring specimen containers, such as vials with caps, between storage cassettes and carrier cassettes. The storage cassettes are designed for cryogenic storage, while carrier cassettes are temporarily placed in portable carriers. The system reads identification data from wireless transponders, verifies container presence and position, provides visual mappings, and issues alerts and corrective commands in case of inconsistencies. It also enables detailed specimen inventory tracking.

### BRIEF SUMMARY

Accordingly, it is desirable to provide new transfer stations, interrogation stations, and related methods for transferring biological specimens (*e.g.,* eggs, sperm, embryos) between a cryogenic freezer, which is typically a large, heavy piece of equipment that is often stationary, and a portable, thermally insulated, carrier, for example for eventual transport to a worksite or workstation at which work will be performed (fertilization, implantation). It is also desirable to provide new transfer stations, interrogation stations, and related methods to facilitate identification of stored biological specimens or samples and evidence chain-of-custody during transfer to and from relatively long term (*e.g.,* days, months, years) cryogenic storage. These objectives are achieved with an interrogation station according to claim 1 and a method in accordance with claim 10, respectively.

An interrogation station facilitates transfer of specimen containers (*e.g.,* vials with caps) into and out of a cryogenic freezer (also known as a dewar). A plurality of the specimen containers may be carried by a storage cassette, which may be designed to be stored in the cryogenic freezer, or in a specimen transporter, which is designed to carry the specimen containers from one location to another while maintaining the viability of the biological specimen within the specimen containers (e.g., by maintaining a cryogenic environment within the specimen transporter that surrounds the specimen containers).

Both the storage cassette and the specimen transporter may be positioned at the interrogation station while one or more of the specimen containers are transferred from one of the storage cassette and the specimen transporter to the other of the storage cassette and the specimen transporter. Identification information is read from wireless transponders carried by the specimen containers, and optionally carried by the storage cassette and the specimen transporter. Inventories of specimen containers and even specific specimen holders may be maintained, as or with proof of chain of custody. Automated storage and retrieval may be provided, for instance via robotic appendages and/or conveyors.

The interrogation station includes a frame and a well, and an array of antennas positioned beneath a bottom of the well, spaced at a nominal distance, within a defined tolerance, from the bottom of the well, and laterally aligned with opening in the frame that provide access to the well. The opening(s) of the frame and/or well may be sized to removably receive buckets, the buckets defining reservoirs, for example to hold carriers of specimen containers and/or cryogenic fluid. The frame and/or well may include one or more features (*e.g.,* spacing features and/or registration features), which physically engage complementary features (*e.g.,* spacing features and/or registration features) on the buckets, to position the buckets with respect to the bottom of the well in a vertical direction as well as laterally, thereby vertically spacing and laterally aligning the bucket with the antennas of the array of antennas. Each bucket includes one or more features (*e.g.,* spacing features and/or registration features) which physically engage complementary features (*e.g.,* spacing features and/or registration features) on the carriers (*e.g.,* bulk carriers), to position the carriers with respect to the buckets in a vertical direction as well as laterally, thereby vertically spacing and laterally aligning the carriers with the antennas of the array of antennas. Each carrier includes a cassette with an array of through holes, sized to hold respective specimen containers at fixed locations in the carrier. Each carrier includes one or more features (*e.g.,* spacing features and/or registration features) which physically engage complementary features (*e.g.,* spacing features and/or registration features) on the cassette (*e.g.,* bulk carriers), to position the cassettes with respect to the carriers in a vertical direction as well as laterally, thereby vertically spacing and laterally aligning the cassettes and specimen containers with their attached RFID transponders with the antennas of the array of antennas of the RFID interrogator.

The well may, for example, advantageously provide a thermal barrier (*e.g.,* air gap) between a cryogenic fluid (*e.g.,* liquid nitrogen) and a circuit board and/or electronics of the RFID interrogator, providing thermal protection to the circuit board and/or electronics. The well may optionally safely collect any cryogenic fluid that may spill, for example from buckets or carriers, into the well.

The interrogation station may be formed or provided as an integral unit (*e.g.,* drop in unit), which can be installed in an opening in a transfer surface of any instances of a transfer station, for example where the instances of the transfer station are of a same make and model as one another, or where the instances of the transfer station are of different makes and/or models from one another. As explained herein, the interrogation stations inherently positions antennas of an interrogator (*e.g.,* RFID interrogator) to be laterally aligned with respective locations at which transponders on specimen containers will be positioned and at nominal perpendicular distances, within a specified tolerance, from those locations. This advantageously allows transfer stations to be easily outfitted with interrogation stations or updated interrogation stations, without requiring expensive and painstaking efforts to align and space the antennas relative to the transfer surface of the transfer station.

### BRIEF DESCRIPTION OF THE DRAWINGS

In the drawings, identical reference numbers identify similar elements or acts. The sizes and relative positions of elements in the drawings are not necessarily drawn to scale. For example, the shapes of various elements and angles are not drawn to scale, and some of these elements are arbitrarily enlarged and positioned to improve drawing legibility. Further, the particular shapes of the elements as drawn are not intended to convey any information regarding the actual shape of the particular elements, and have been solely selected for ease of recognition in the drawings.
Figure 1 is a front, top, isometric view of a cryogenic storage system including a cryogenic storage tank that holds a coolant, a robotic transfer system operable to transfer biological specimen into and out of the cryogenic storage tank, and a transfer station including an interrogation station to handle and track the transfer of biological specimens into and out of the cryogenic freezer and to maintain a chain of custody for the biological specimens, according to at least one illustrated embodiment.
Figure 2 is a top, isometric view of the interrogation station illustrated in Figure 1, according to one embodiment.
Figure 3 is a bottom, isometric view of the interrogation station illustrated in Figure 2.
Figure 4 is a top, plan view of the interrogation station illustrated in Figure 2.
Figure 5 is a front, elevation view of the interrogation station illustrated in Figure 2.
Figure 6 is a side, elevation view of the interrogation station illustrated in Figure 2.
Figure 7 is a partially exploded, isometric view of the interrogation station illustrated in Figure 2.
Figure 8 is a top, isometric view of a portion of the interrogation station illustrated in Figure 2.
Figure 9 is a bottom, isometric view of the portion of the interrogation station illustrated in Figure 8.
Figure 10 is an exploded view of the portion of the interrogation station illustrated in Figure 8.
Figure 11 is a top, isometric view of a specimen container, according to one embodiment.
Figure 12 is a bottom, isometric view of the specimen container illustrated in Figure 11.
Figure 13 is an exploded view of the specimen container illustrated in Figure 11.
Figure 14 is an isometric view of a storage cassette supporting a specimen container, according to one embodiment.
Figure 15 is an isometric, exploded view of the storage cassette illustrated in Figure 14.
Figure 16 is a cross-sectional view of the storage cassette illustrated in Figure 14 along line A-A.
Figure 17 is an isometric view of the storage cassette illustrated in Figure 14 with an attached handle.
Figure 18 is an isometric view of the handle illustrated in Figure 17.
Figure 19 is an isometric view of a specimen transporter, according to one embodiment.
Figure 20 is an isometric, exploded view of the specimen transporter illustrated in Figure 19.
Figure 21 is a cross-sectional view of the specimen transporter illustrated in Figure 19 along line B-B, supporting a specimen container.
Figure 22 is a top plan view of a bucket of the interrogation station, according to one embodiment.
Figure 23 is an isometric view of the interrogation station illustrated in Figure 2 with one of the buckets illustrated in Figure 22 in a seated configuration and another of the buckets illustrated in Figure 22 in an unseated configuration.
Figure 24 is a cross-sectional view of the interrogation station illustrated in Figure 2, supporting the storage cassette illustrated in Figure 14 and the specimen transporter illustrated in Figure 19, each carrying one of the specimen containers illustrated in Figure 11.
Figure 25 is a functional block diagram of a processor-based transfer station of a cryogenic storage system, according to at least one embodiment.
Figure 26 is a first portion of a flow diagram showing a method of operation in a specimen transfer system to transfer specimen containers that hold biological specimens from positions in a storage cassette, which can be stored in a cryogenic freezer, to positions in a portable thermally insulated specimen transporter, according to at least one embodiment.
Figure 27 is a second portion of the flow diagram illustrated in Figure 26.
Figure 28 is a first portion of a flow diagram showing a method of operation in a transfer system to transfer specimen containers that hold biological specimens from positions in a portable thermally specimen transporter, to positions in a storage cassette, which can be stored in a cryogenic freezer, according to at least one embodiment.
Figure 29 is a second portion of the flow diagram illustrated in Figure 28.

### DETAILED DESCRIPTION

In the following description, certain specific details are set forth in order to provide a thorough understanding of various disclosed embodiments. However, one skilled in the relevant art will recognize that embodiments may be practiced without one or more of these specific details, or with other methods, components, materials, etc. In other instances, well-known structures associated with computer systems, actuator systems, and/or communications networks have not been shown or described in detail to avoid unnecessarily obscuring descriptions of the embodiments.

Unless the context requires otherwise, throughout the specification and claims which follow, the word "comprise" and variations thereof, such as, "comprises" and "comprising" are to be construed in an open, inclusive sense, that is as "including, but not limited to."

Reference throughout this specification to "one implementation" or "an implementation" or to "one embodiment" or "an embodiment" means that a particular feature, structure or characteristic described in connection with the implementation or embodiment is included in at least one implementation of embodiment. Thus, the appearances of the phrases "in one implementation" or "in an implementation" or "in one embodiment" or "in an embodiment" in various places throughout this specification are not necessarily all referring to the same implementation or embodiment. Furthermore, the particular features, structures, or characteristics may be combined in any suitable manner in one or more implementations or embodiments, provided such combinations fall within the scope of the claims.

As used in this specification and the appended claims, the singular forms "a," "an," and "the" include plural referents unless the content clearly dictates otherwise. It should also be noted that the term "or" is generally employed in its sense including "and/or" unless the content clearly dictates otherwise.

The headings and Abstract of the Disclosure provided herein are for convenience only and do not interpret the scope or meaning of the embodiments.

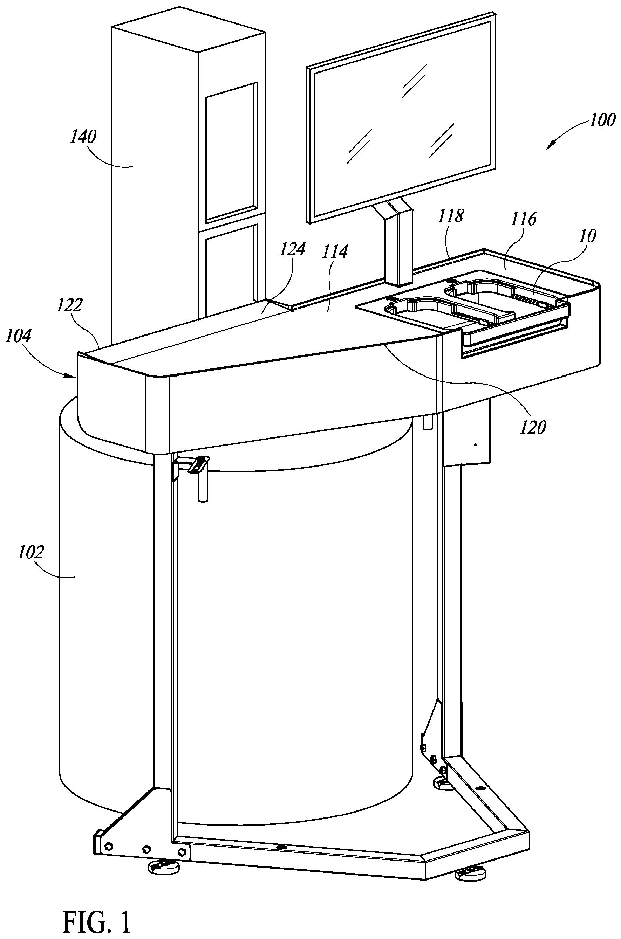

Referring to Figure 1, a cryogenic storage system 100 may take a large variety of forms, typically including cryogenic freezer 102 which can store specimen containers in a cryogenic environment, for example immersed in a bath of liquid nitrogen at a temperature at or below about negative 190°C. The cryogenic freezer 102 is typically highly thermally insulated, and may include stainless steel interior and exterior walls with a vacuum and/or other thermal insulating material therebetween.

The cryogenic storage system 100 may include a transfer station 104 to facilitate the transfer of biological specimen to and from the cryogenic freezer 102, including identification of said transferred biological specimen and evidence chain-of-custody during handling of said stored biological specimen. According to one implementation, the transfer station 104 may be located adjacent, for example immediately adjacent, the cryogenic freezer 102. The transfer station 104 may include a transfer surface 114 having a planar portion 116 and a peripheral wall 118 that extends upwardly from the planar portion 116 about at least a portion of a periphery 120 of the transfer surface 114. The transfer station 104 may include an interrogation station 10 that facilitates the transfer of biological specimen from one container to another container. The interrogation station 10 may, for example, be at least partially recessed into the transfer surface 114, allowing cold (*e.g.,* liquid nitrogen bath) temporary holding of biological specimens while outside of the cryogenic freezer 102, for example during transfer operations (*e.g.,* retrieval from and/or placement in cryogenic freezer 102). The transfer station 104 may include an elevated floor, which facilitates a shorter distance to a top of the cryogenic freezer 102 for a user standing on the elevated floor as opposed to standing on a non-elevated floor (e.g. the floor upon which the cryogenic freezer 102 rests).

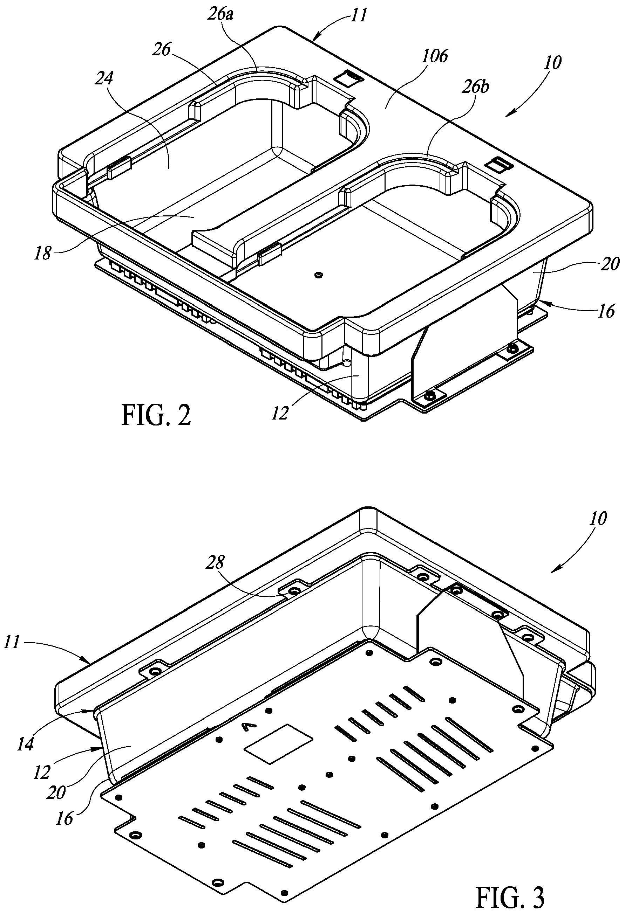

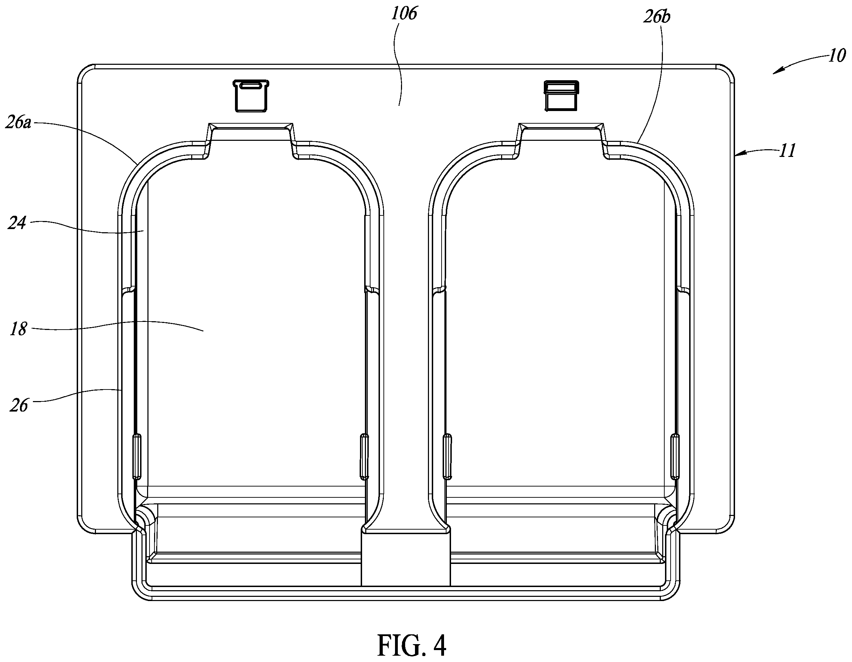

Referring to Figures 2 to 12, the cryogenic storage system 100 may include an interrogation station 10 that facilitates the transfer of biological specimen from one container to another container. The interrogation station 10 may maintain a suitable temperature for the biological specimen during the transfer process. Additionally, the interrogation station 10 may identify and track each biological specimen during the transfer process. The interrogation station 10 may be formed or provided as an integral unit (*e.g.,* drop in unit), which can be installed in an opening in a transfer surface 114 of any instances of a transfer station 104, for example where the instances of the transfer station 104 are of a same make and model as one another, or where the instances of the transfer station 104 are of different makes and/or models from one another. As explained herein, the interrogation stations 10 inherently positions antennas of an interrogator (*e.g.,* RFID interrogator) to be laterally aligned with respective locations at which transponders on specimen containers will be positioned and at nominal perpendicular distances, within a specified tolerance, from those locations. This advantageously allows transfer stations 104 to be easily outfitted with interrogation stations 10 or updated interrogation stations 10, without requiring expensive and painstaking efforts to align and space the antennas relative to the transfer surface 114 of the transfer station 104.

Referring to Figures 2 to 7, the interrogation station 10 includes a frame 11 and a well 12 (one illustrated). The well 12 includes an upper portion 14 and a lower portion 16. The lower portion 16 includes a bottom 18 and at least one sidewall 20 that extends upwardly from the bottom 18. The upper portion 14 of the well 12 includes an open top 22, wherein the bottom 18, the at least one sidewall 20, and the top 22 cooperatively delineate an interior cavity 24 of the well 12.

The frame 11 includes at least one opening 26a, 26b (two shown) extending through an upper surface 106 of the frame to the open top 22 of the well 12 so as to provide entry and access to the interior cavity 24. As illustrated, the frame 11 include a first opening 26a and a second opening 26b. The first opening 26a and the second opening 26b may be identical in size and shape, as shown in the illustrated embodiment. Alternatively, the first opening 26a and the second opening 26b may have a different size, shape, or both size and shape. As explained herein, the well 12 and the openings 26a, 26b of the frame 11 are sized to removably receive and hold buckets, which in turn hold carriers (*e.g.,* bulk carriers) which may be immersed or partially immersed in a coolant (*e.g.,* nitrogen in liquid and/or vapor form). The well 12 may, for example, advantageously provide a thermal barrier (*e.g.,* air gap) between a cryogenic fluid (*e.g.,* liquid nitrogen) and a circuit board and electronics, and optionally safely collect any cryogenic fluid that may spill, for example from buckets or carriers, into the well 12.

The frame 14 and the well 12 may be manufactured as two or more separate structures that are joined together (e*.g.,* by one or more fasteners 28). Alternatively, the frame 14 and the well 12 may be formed as one monolithic piece. The well 12 may comprise, or even consist of, a material with a wide operating temperature range, that is resistant to damage caused by exposure to repeated temperature changes *(e.g.,* caused by intermittent exposure or proximity to coolant, for example cryogenic fluids such as liquid nitrogen, and associated cold temperatures). The material of the well 12 may also be precisely machinable with adherence to tight tolerances. According to at least one implementation, the well 12 is made from an engineering thermoplastic, such as polyoxymethylene (also known as Delrin).

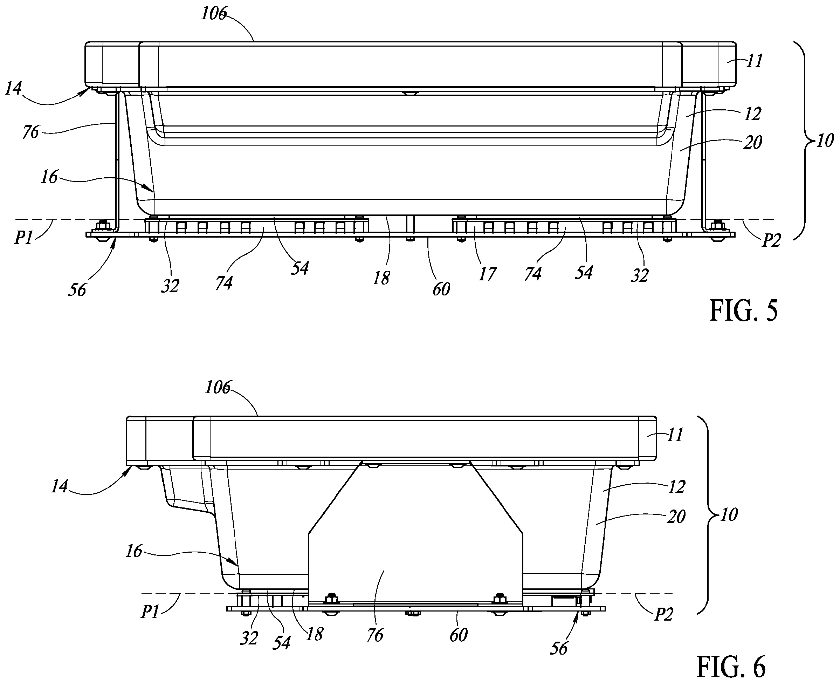

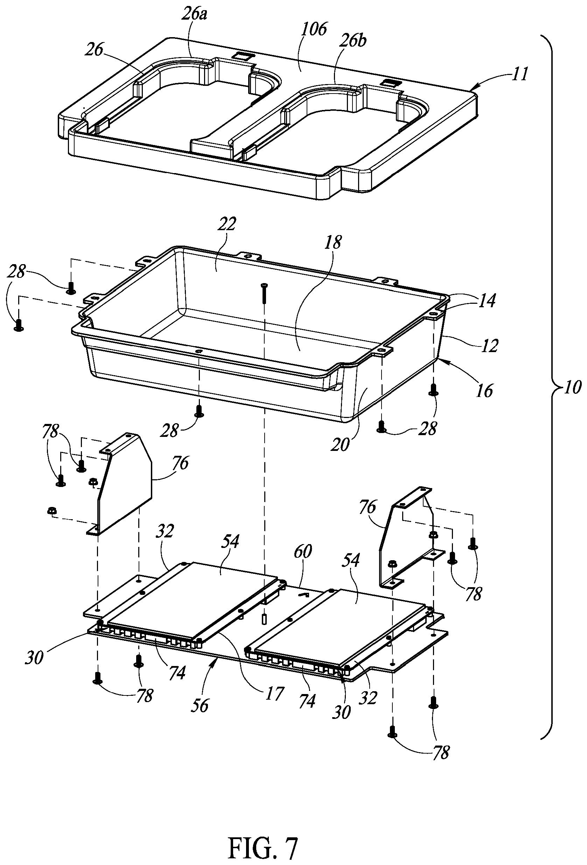

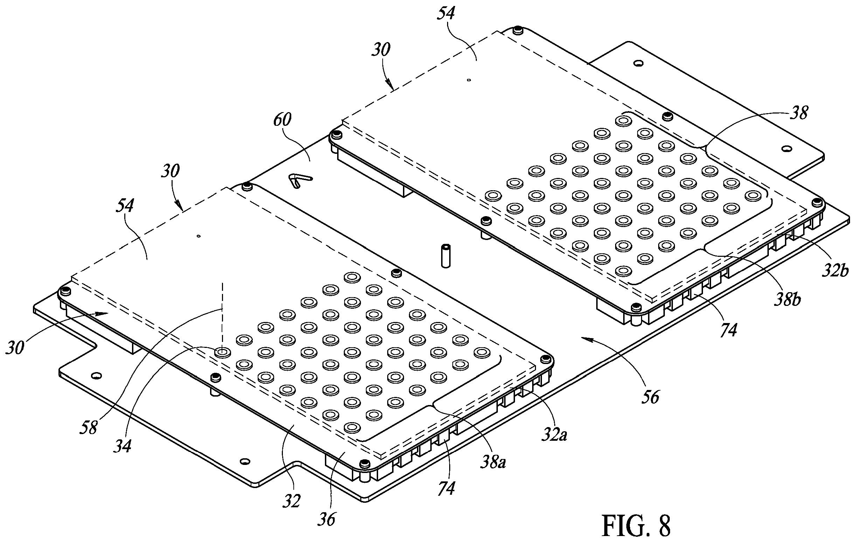

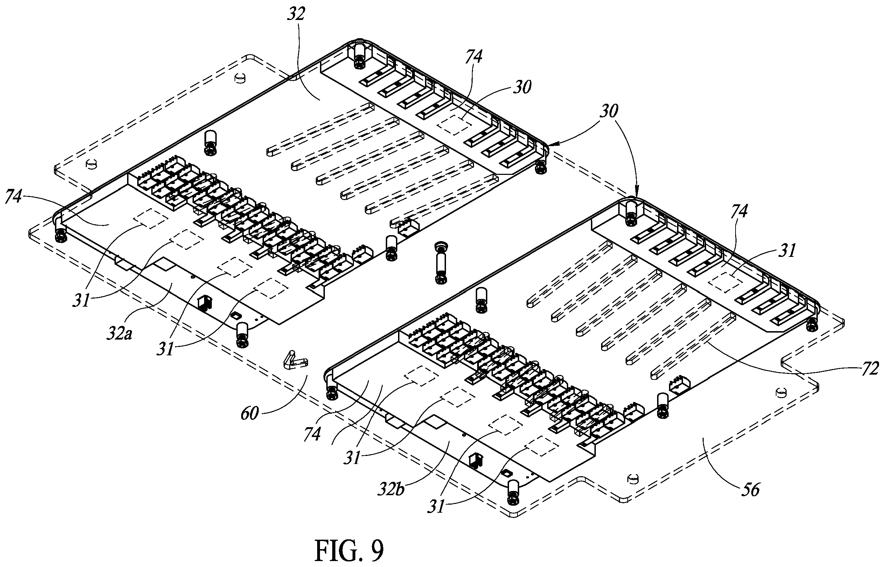

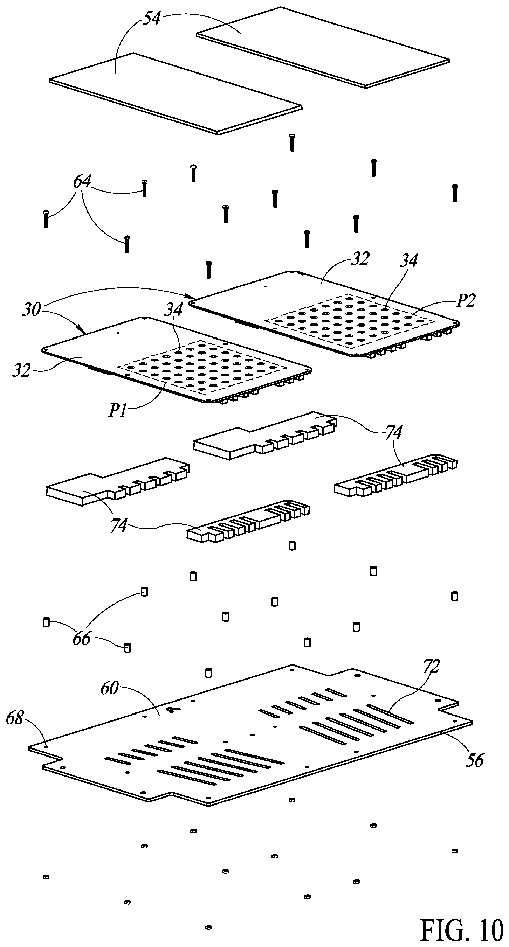

Referring to Figures 5 to 10, the interrogation station 10 may further include at least one sensor capable of identifying individual specimen containers. According to at least one implementation, the at least one sensor includes an RFID reader 30 (also known as an interrogator, called out in Figures 7-10), including for example one or more RFID reader integrated circuits 31 (illustrated in broken line in Figure 9) and antennas 34 (one called out in Figures 8 and 10), for instance carried by one or more printed circuit boards 32. The RFID reader 30 may optionally also include one or more processors, for example, one or more of: one or more microcontrollers, one or more microprocessors, one or more central processing units, one or more digital signal processors (DSPs), one or more graphics processing units (GPUs), one or more application specific integrated circuits (ASICs), one or more field programmable gate arrays (FPGAs), and/or one or more programmable logic controllers (PLCs). The RFID reader 30 may each include one or more nontransitory storage media, for example, one or more nonvolatile storage media and/or one or more volatile storage media, for example one or more of: one or more read only memories (ROMs), one or more random access memories (RAMs), one or more FLASH memory, one or more magnetic disk drives, one or more optical disk drives, one or more solid state drives, one or more cache memories, and/or one or more registers of one or more processors.

As shown, the interrogation station 10 includes at least one printed circuit board 32 (e.g., mounted beneath the well 12 as described in greater detail below). The RFID reader 30 may include at least one antenna 34 positioned beneath the well 12 (*e.g.,* mounted to an upper side 36 of the printed circuit board 32). According to at least one implementation, the RFID reader 30 may include at least one array 38 of the antennas 34 and at least one RFID interrogation circuit or radio (*e.g.,* integrated circuit RFID readers 1400a-1400j), for example mounted to or otherwise carried by the printed circuit board 32.

As shown, the at least one array 38 of the antennas 34 may include a two-dimensional array of, for example, coil antennas, and the two-dimensional array may include, for example, up to a nine-by-nine (9x9) array of the antennas 34. Optionally, the antennas 34 may be arranged as one-dimensional arrays.

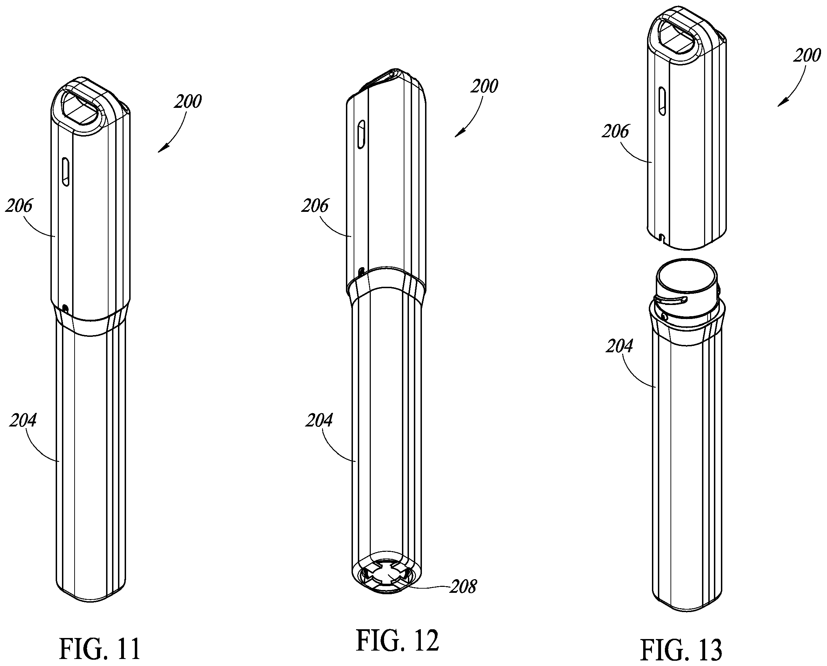

The RFID reader 30 may perform wireless interrogation of wireless transponders, (*e.g.,* passive radio frequency identification (RFID) transponders) used to tag respective specimen containers (*e.g.,* the specimen containers 200 shown and described in reference to Figures 11 to 13 below). The RFID reader 30 may individually identify the specimen containers 200 via unique identifiers stored by wireless transponders, and locate a respective position of the specimen containers 200 in an arrangement of the specimen containers 200.

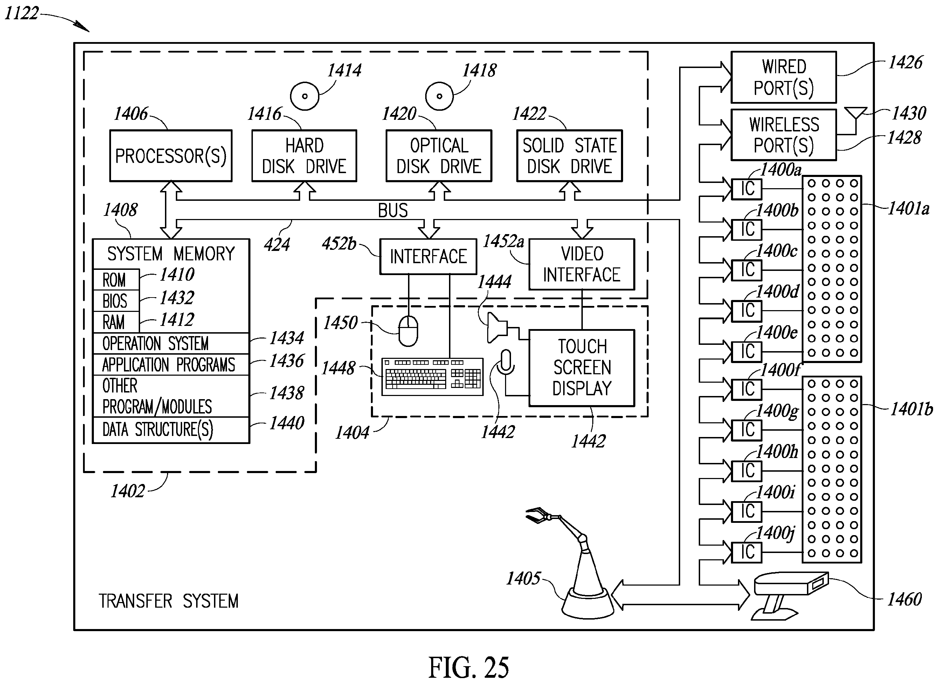

As shown, the interrogation station 10 may include a first two-dimensional array 38a of the antennas 34 and a second two-dimensional array 38b of the antennas 34. The interrogation station 10 may include a first printed circuit board 32a that carries the first two-dimensional array 38a and a second printed circuit board 32b that carries the second two-dimensional array 38b. By virtue of the layout of the first and second arrays of antennas 38a, 38b and the alignment features of the components of the interrogation station 10 (described in detail below), each of the antennas 34 is positioned to align with a respective position in which a respective wireless transponder of a specimen container 200 may be located when the interrogation station 10 is used as described below. The RFID reader 30 may include at least one radio (*e.g.,* integrated circuit RFID readers 1400a-1400j illustrated in Figure 25) that causes transmission of interrogation signals via the antennas 34 and which may decode unique identifiers from response signals returned by the wireless transponders in response to the interrogation signals.

As shown in Figures 5 and 6, the printed circuit board 32 may be mounted beneath the well 12 such that at least a portion of the printed circuit board 32 is accessible from the surrounding environment, (*i.e.,* not enclosed within an enclosure). Accessibility to the surrounding environment may result in increased cooling of the printed circuit board 32 and their associated electronic components, which tend to heat up during operation. Thus, while it may seem beneficial to enclose the printed circuit board 32 to isolate the printed circuit board 32 from the moisture and cold temperatures resultant from coolant (*e.g.,* cryogenic fluid) supported by the well 12 within close proximity to the printed circuit board 32, the excess heat generated within the enclosure may instead have a detrimental effect. Additionally, the proximity of the enclosure to the extreme low temperature resultant from the coolant (*e.g.,* cryogenic fluid) can result in increased moisture formation within the enclosure as opposed to an open support frame. Further, enclosing the printed circuit board 32 can subject the printed circuit board 32, the electrical and electronic components carried thereby, and various electrical connections (*e.g.,* solder) therebetween, to more extreme thermally cycling, increasing the risk of delamination of the printed circuit board 32, failure of one or more components and/or electrical connections.

Alternatively, according to at least one implementation, the interrogation station 10 may include an enclosure that receives the printed circuit board 32 carrying the at least one array 38 of antennas 34. Active and/or passive cooling (*e.g.,* fans, vents, etc.) may be provided to remove heat from the enclosure generated by operation of components of the printed circuit board 32.

The printed circuit board 32 may include additional components such as batteries, resistors, LEDs, transistors, capacitors, etc. that enable functionality of the RFID reader 30. As certain components of the RFID reader 30 are susceptible to temperature, moisture or both, the interrogation station 10 may include features that protect those susceptible components, as they are operating in close proximity to the coolant, which tends to generate extreme temperatures and excess moisture in its proximity.

According to at least one implementation, the printed circuit board 32 may be treated to provide increased resistance to damage due to exposure to extremely cold temperatures. The printed circuit board 32 may be treated with a parylene vapor deposition process, and then dipped in polyurethane to prevent delamination.

According to at least one implementation, the interrogation station 10 includes an insulator 54. The insulator 54 may be positioned between the printed circuit board 32 and the well 12. As shown, the insulator 54 may directly contact both the printed circuit board 32 and the well 12. The insulator 54 may be secured *(e.g.,* by an adhesive, a fastener, etc.) relative to one or both of the printed circuit board 32 and the bucket housing 12. Alternatively, the insulator 54 may be held in place by a friction fit with both of the printed circuit board 32 and the well 12.

The insulator 54 may be deformable (*e.g.,* a foam or a foam-like material) such that pressure applied to the insulator 54 towards the printed circuit board 32 causes the insulator 54 to deform and partially surround components on the upper side 36 of the printed circuit board 32, (*e.g.,* the antennas 34). The insulator 54 may thereby protect the antennas 34 from exposure to extremely low temperatures, resultant from the proximity of the coolant, that may otherwise affect the performance and/or the functionality of the antennas 34. The insulator 54 may include a hydrophobic material to repel moisture from the partially surrounded components. According to one embodiment, the insulator 54 includes an aerogel. Note that in Figure 8, the insulator 54 (including two separate portions), is shown in dashed lines and as being transparent to allow visibility of the antennas 34.

Proper alignment of the antennas 34 and the well 12 is important for accurate identification of specimen being transferred with the aid of the interrogation station 10. Accordingly, the interrogation station 10 includes a coupler 56 that secures the printed circuit board 32 relative to the well 12. As shown the coupler 56 secures the printed circuit board 32 relative to the well 12 such that the array 38 of the antennas 34 are laterally aligned with the opening 26 in the frame 11 and the open top 22 of the well 12, as well as being spaced perpendicularly or vertically from the bottom of the well 12 by a nominal distance (*e.g.,* 5 mm) within a specified tolerance (*e.g.,* +/-10%).

According to at least one implementation, the array 38 of the antennas 34 is located within a plane P1. The coupler 56 secures the printed circuit board 32 relative to the well 12 and the frame 11. For example, the coupler secures the printed circuit board 32 to longitudinally position the array 38 of the antennas 34 at a defined nominal distance (*e.g.,* 5 mm) within a specified tolerance (*e.g.,* +/- 10%) of the bottom of the well 12 along a direction normal to the plane P1 (*e.g.,* a vertical direction indicated by arrow J). Also for example, the coupler secures the printed circuit board 32 to laterally position the array 38 of the antennas 34 laterally aligned or in registration with the opening(s) 26 in the frame 11 and the open top 22 of the well 12, for instance along a first lateral direction and a second lateral direction, the first and second lateral directions perpendicular to the vertical direction and also perpendicular to one another.

According to at least one implementation, the antennas 34 within the array 38 each include a respective beam axis 58, and the coupler 56 secures the printed circuit board 32 relative to the well 12 such that the beam axes 58 of the antennas 34 in the array 38 are respectively laterally aligned with a respective location at which an antenna of an RFID transponder attached to a vial will be located when a bulk carrier is positioned in a bucket in the opening of the frame 11 and in the well 12. The beam axis 58 may include an axis of maximum radiation (*e.g.,* the axis passing through the center of the main lobe of the radiation plot of the antenna). According to at least one implementation, the antenna 34 may be a coil antenna or a helical antenna and the beam axis 58 may be collinear with a central axis of the coil antenna or the helical antenna.

As shown in the illustrated implementation, the coupler 56 includes a plate 60 that supports the printed circuit board 32. The plate 60 supports the printed circuit board 32 such that there is a standoff 17, or gap, between the plate 60 and the printed circuit board 32. The standoff 17 may be sized to provide adequate space for components of the printed circuit board 32 mounted on a lower side 62 of the printed circuit board 32 (the lower side 62 being opposite the upper side 36). Fasteners 64 with a spacer 66 may be used to secure the plate 60 to the printed circuit board 32, with the spacer 66 being positioned between the plate 60 and the printed circuit board 32, such that a height of the spacer 66 sets a height of the standoff 17. According to at least one implementation, the height of the standoff 17 between the plate 60 and the printed circuit board 32 is between 5 mm and 15 mm, for example 8 mm.

According to at least one implementation, the plate 60 may be thin (*e.g.,* having a thickness less than the standoff 17, or having a thickness similar to that of the printed circuit board 32. The plate 60 may be stiff (*e.g.,* stiffer than the printed circuit board 32) such that the plate 60 provides additional strength to the printed circuit board 32 when the printed circuit board 32 is secured to the plate 60. The plate 60 may be transparent so that the components of the printed circuit board 32 that are mounted to the lower side 62 of the printed circuit board 32 are visible through the plate 60. The components of the printed circuit board 32 may include lights or other indicators, and the plate 60 being transparent allows the lights or indicators to be viewed without disconnecting the plate 60 from the printed circuit board 32.

The plate 60 may include through holes 68 extending through the thickness of the plate 60. Some of the through holes 68 may receive respective ones of the fastener 64 used to secure the plate 60 to the printed circuit board 32. These fastener through holes 70 may be circular, as shown in the illustrated embodiment. Others of the through holes 68 may provide access through the plate 60 to the standoff 17. These other through holes (hereinafter vent through holes 72) may act as vents that provide a path for heat generated by the components of the lower side 62 of the printed circuit board 32 to exit the standoff 17. The vent through holes 72 may have various shapes, (*e.g.,* slots as shown in the illustrated implementation).

Different ones of the vent through holes 72 may have different shapes and sizes, and the vent through holes 72 may be positioned to align with certain components of the printed circuit board 32 that produce more heat (*e.g.,* resistors, power supplies or converters, integrated circuit RFID readers or radios). Further, the vent through holes 72 may be positioned so as to be out of alignment with certain components of the printed circuit board 32 that are susceptible to moisture. Note that in Figure 9 the plate 60 is shown in dashed lines and as being transparent to allow visibility of the underlying components of the circuit board 32.

According to one embodiment, the interrogation station 10 may include a hydrophobic material 74 positioned between one or more portions of the lower side 62 of the printed circuit board 32 and the plate 60. The hydrophobic material 74 may be deformable (*e.g.,* a foam or a foam-like material) with a height slightly greater than that of the standoff 17. Thus, when securing the printed circuit board 32 to the plate 60 the hydrophobic material 74 may be compressed and secured between the printed circuit board 32 and the plate 60 via a friction fit. According to one embodiment, the height of the hydrophobic material 74 is greater than the height of the insulator 54. Alternatively, other connections (e.g., fasteners, adhesives, etc.) may be used to secure the hydrophobic material 74 within the standoff 17.

The hydrophobic material 74 may be similar to the insulator 54 such that pressure applied to the hydrophobic material 74 towards the printed circuit board 32 causes the hydrophobic material 74 to deform and partially surround components on the lower side 62 of the printed circuit board 32, (*e.g.,* the relays). According to at least one implementation, the hydrophobic material 74 is an aerogel, for instance a silica aerogel wrap, for instance having an 8 mm thickness.

Referring still to Figures 5 to 10, the coupler 56 may further include one or more brackets 76 (two shown) that secures the plate 60 relative to the well 12 and frame 11. As shown, the bracket 76 may support the plate 60 such that the antennas 34 on the upper side 36 of the printed circuit board 32 are in close proximity to an outer surface of the bottom 18 of the well 12. According to at least one implementation, the antennas 34 on the upper side 36 of the printed circuit board 32 are less than 10 mm from the outer surface of the bottom 18 of the well 12. According to at least one implementation, the antennas 34 on the upper side 36 of the printed circuit board 32 are less than 5 mm from the outer surface of the bottom 18 of the well 12. According to at least one implementation, the antennas 34 on the upper side 36 of the printed circuit board 32 are no more than the thickness of the insulator 54 from the outer surface of the bottom 18 of the well 12.

Fasteners (*e.g.,* additional ones of the fasteners 64, or fasteners 78 with a different size and/or shape, for instance threaded fasteners including screws, bolts and nuts, or other fasteners including clamps, pins, rivets, nails, etc.) may be used to secure the bracket 76 to the well 12 (*e.g.,* an underside of a flange at the open top 22 of well 12), or alternatively to under the top surface 106 of the frame 11, and to secure the bracket(s) 76 to the plate 60. As shown, the bracket(s) 76 may be secured to the well 12 or frame 11 such that the fasteners 78 are not visible (i.e., the fasteners 78 do not protrude through) an upper surface of the top 22 of the frame 11.

Referring to Figures 1 and 11 to 13, the cryogenic storage system 100 may include a specimen container 200, which is typically stored for long term storage in a cryogenic environment (*e.g.,* inside the cryogenic freezer 102). Each of the specimen containers 200 may include a vial 204, a cap 206, one or more wireless transponders (*e.g.,* radio frequency identification (RFID) transponders) 208, and an elongated specimen holder *(e.g.,* straw, rod, spatula). Additionally, the specimen containers 200 may include one or more machine-readable symbols. The specimen containers 200, according to one example, may store specimens of biological tissue, for instance eggs, sperm or embryos. Various implementations of specimen containers are described in U.S. patent application 62/900,281, filed September 13, 2019; U.S. patent application 62/880,786, filed July 31, 2019; U.S. patent application 62/879,160, filed July 26, 2019; U.S. patent application 62/741,986, filed October 5, 2018; and U.S. patent application 62/741,998, filed October 5, 2018, the disclosures of which are hereby incorporated by reference.

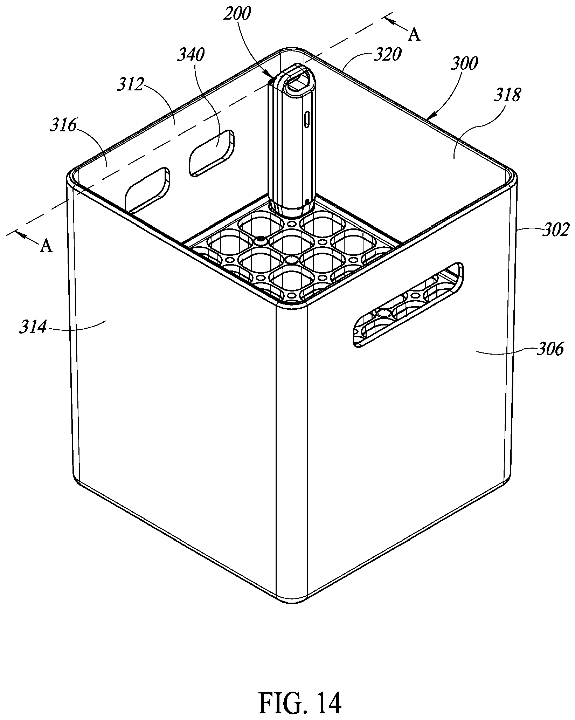

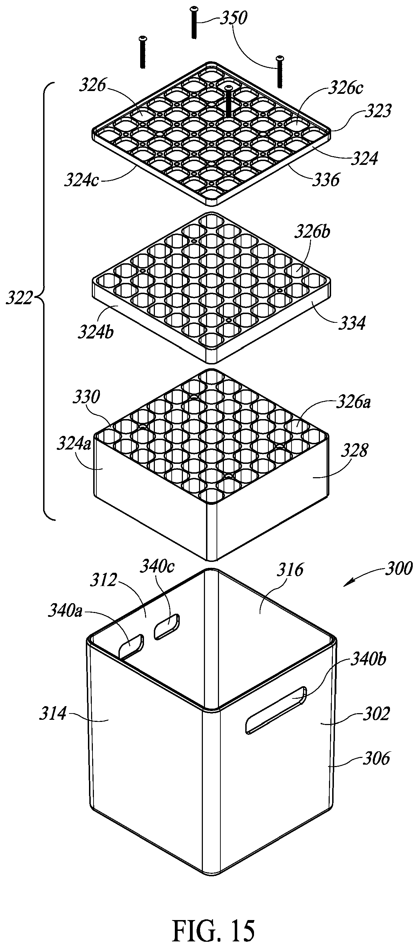

Referring to Figures 14 to 16, one or more of the specimen containers 200 may be carried by a bulk carrier, for example a storage cassette 300, of the cryogenic storage system 100, for example arrayed in a two-dimensional array (*e.g.,* 7x7, 10x10, 8x12, 14x14). The storage cassette 300 may be designed to remain in the cryogenic refrigerator except for brief periods when removal is needed to retrieve a specimen within one of the specimen containers 200 carried by the storage cassette 300.

The storage cassette 300 maintains cryogenic conditions for an array of the specimen containers 200, for example 49 separate ones of the specimen containers 200, according to at least one implementation. As shown in the illustrated embodiment, the storage cassette 300 may include a housing 302 having a bottom 304 and at least one sidewall 306. The bottom 304 may have an inner facing surface 308 and an outer facing surface 310. The at least one sidewall 306 may have an inner facing surface 312 and an outer facing surface 314.

The inner facing surface 308 of the bottom 304 and the inner facing surface 312 of the at least one sidewall 306 may delineate an interior compartment 316 of the housing 302. The interior compartment 316 may have an interior compartment profile, and the housing 302 may have an opening 318 at a top 320 thereof, formed by the top 320, which is opposite the bottom 304 with respect to a vertical dimension, as shown in the illustrated embodiment.

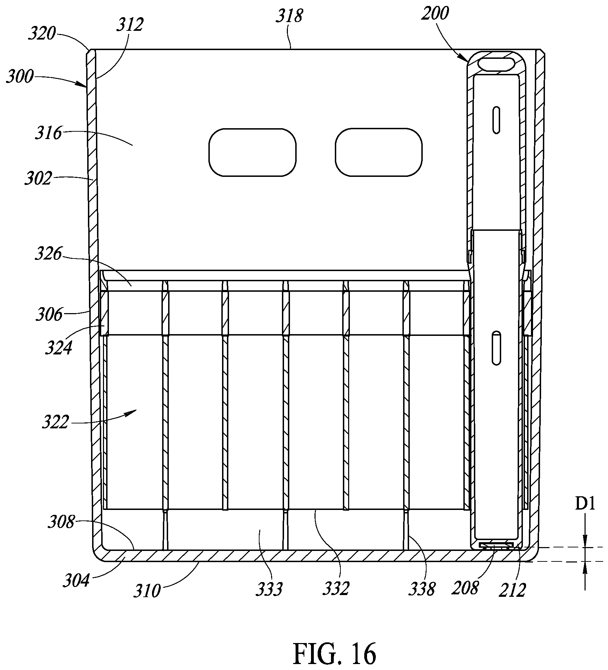

The storage cassette 300 may further include a tray 322 that supports one or more of the specimen containers 200 within the interior compartment 316 of the housing 302. The tray 322 may include an outer perimeter 323 that corresponds to the inner facing surface 312 of the at least one sidewall 306 such that the tray 322 is insertable into the interior compartment 316, and once inserted relative movement of the housing 302 and the tray 322 is inhibited. According to at least one implementation, the storage cassette 300 includes a seated configuration (*e.g.,* as shown in Figure 16) in which the tray 322 abuts the housing 302 such that further vertical movement of the tray 322 towards the bottom 304 of the housing 302 is prevented, and lateral movement of the tray 322 relative to the housing 302 is also prevented. In the seated configuration, vertical movement of the tray 322 away from the bottom 304, and towards the opening 318 at the top 320 of the housing 302 may not be inhibited, thereby allowing removal of the tray 322 from the interior compartment 316.

The tray 322 includes a substrate 324 and one or more (*e.g.,* a plurality of) through holes 326 extending therethrough. The through holes 326 may each be sized and shaped to correspond to one of the specimen containers 200. According to at least one implementation, the through holes 326 correspond to the specimen containers 200 such that when one of the specimen containers 200 is inserted into one of the through holes 326 relative movement of the specimen container 200 and the tray 322 is inhibited.

The tray 322, according to at least one implementation, may include a plurality of substrates (*e.g.,* the substrate 324, referred to herein as a first substrate 324a, a second substrate 324b, and a third substrate 324c). As shown, each of the plurality of substrates may include a respective plurality of through holes (*e.g.,* a first plurality of through holes 326a, a second plurality of through holes 326b, and a third plurality of through holes 326c. The plurality of substrates may correspond to one another such that each of the first plurality of through holes 326a, the second plurality of through holes 326b, and the third plurality of through holes 326c are aligned to receive one of the specimen containers 200 therein.

According to at least one implementation, the first substrate 324a may optionally be a thermal shunt 328. According to at least one implementation, the optional thermal shunt 328 includes a metal. As shown, the thermal shunt 328 may include a first major face 330 and a second major face 332, the second major face 332 opposed from the first major face 330 across a thickness of the thermal shunt 328. The first plurality of through holes 326a may extend through the thickness of the thermal shunt 328, as shown.

As shown, the thermal shunt 328 may be closely receivable in the interior compartment 316 of the housing 302. The thermal shunt 328 may have an outer profile that is asymmetrical to ensure that the thermal shunt 328 is positioned correctly in the interior compartment 316 of the housing 302. The thermal shunt 328 may be made of any of a variety of materials, preferably having a relatively large thermal mass as compared to the biological materials to be stored in the specimen containers 200 in the storage cassette 300. Suitable materials for the thermal shunt 328 may include, for example, slabs of non-ferrous metals, or metal impregnated polymers where the metal is a non-ferrous metal or the metal is in the form of small pieces, particles or strands that are sufficiently small and discontinuous as to prevent or retard the formation of currents therethrough. In at least some implementations, the thermal shunt 328 takes the form of an aluminum plate, slab, or slug.

The plurality of substrates, for example the second substrate 324b, may further include a thermal insulator 334 closely receivable in the interior compartment 316 of the housing 302. As shown, the thermal insulator 334 may be positioned vertically above the thermal shunt 328 (*e.g.,* abutting the first major surface 330 of the thermal shunt 328) when inside the interior compartment 316). The thermal insulator 334 may reduce heat transfer (via conduction, convection, or both) outward from the thermal shunt 328 toward an external environment of the storage cassette 300 (*e.g.,* out through the opening 318).

According to at least one implementation, the plurality of substrates, for example the third substrate 324c, may include a cover plate 336. As shown, the cover plate 336 and the thermal shunt 328 may be securable to one another, for example with one or more fasteners 350, such that the thermal insulator 334 is captured between the cover plate 336 and the thermal shunt 328. The thermal insulator 334 may include a material that is more pliable than the cover plate 336 and the thermal shunt 328, and thus is less suitable for accepting a fastener. According to at least one implementation, the cover plate 336 and the thermal shunt 328 may each be stiffer than the thermal insulator 334.

Any combination of two or more of the optional thermal shunt 328, the thermal insulator 334, and the cover plate 336 may be stacked in the interior compartment 316 of the housing 302 such that respective ones of each of the first, second, and third plurality through holes 326a, 326b, and 326c are axially aligned (*e.g.,* as shown in Figure 16). The first, second, and third plurality of through holes 326a, 326b, and 326c may have matching shapes, or alternatively, may have different shapes. As shown, the first, second, and third plurality through holes 326a, 326b, and 326c may be square with rounded corners. According to at least one implementation, the first, second, and third plurality through holes 326a, 326b, and 326c may be circular.

The housing 302 may include a number of set offs 338 that extend upwardly from the inner facing surface 308 of the bottom 304 into the interior compartment 316. As shown in the illustrated implementation, the set offs 338 may be positioned to support the tray 322, for example by abutting the second major face 332 of the thermal shunt 328 such that the thermal shunt 328 is spaced from the inner facing surface 308 of the bottom 304 of the housing 302. The spacing of the thermal shunt 328 from the inner facing surface 308 may delineate a volume 333 in which coolant may collect. As shown, the tray 322 may support the specimen container 200 such that a distal portion 212 of the specimen container 200 extends into the volume and is at least partially surrounded by the coolant (if present).

The storage cassette 300 may include a thickness of: the bottom 304, the thermal shunt 328, the thermal insulator 334, the set offs 338, or any combination thereof, such that a portion of the specimen container 200, for example the wireless transponder 208, is within a defined distance D1 of the outer facing surface 310 of the bottom 304. According to at least one implementation, specimen container 200 is supported directly by (*i.e.,* is in direct contact with) the inner facing surface 308, such that the defined distance D1 is equal to a thickness of the bottom 304 of the housing 302 plus an amount (if any) the wireless transponder 208 is recessed into a bottom of the specimen container 200.

The storage cassette 300 may include a thickness of the thermal shunt 328, the set offs 338, or both, such that a specimen 220 within the specimen container 200, is surrounded by the thermal shunt 328 (in the lateral direction) and the thermal shunt 328 is spaced upward and away from the wireless transponder 208.

The housing 302 may include at least one aperture (not shown) in the bottom 304, which permits ingress and egress of coolant into and out of the interior compartment 316 of the housing 302. Alternatively, the bottom 304 may be devoid of any apertures such that coolant may not exit the interior compartment 316 through the bottom 304.

The at least one sidewall 306 may be thermally insulated. The housing 302 may include one or more through apertures 340 formed in the at least one sidewall 306. The one or more apertures 340 may include first and second apertures 340a and 340b opposite one another with respect to a lateral dimension of the housing 302. As shown in the illustrated implementation, the one or more apertures 340 may include adjacent apertures (*e.g.,* the first aperture 340a and a third aperture 340c) formed in one surface of the at least one sidewall 306.

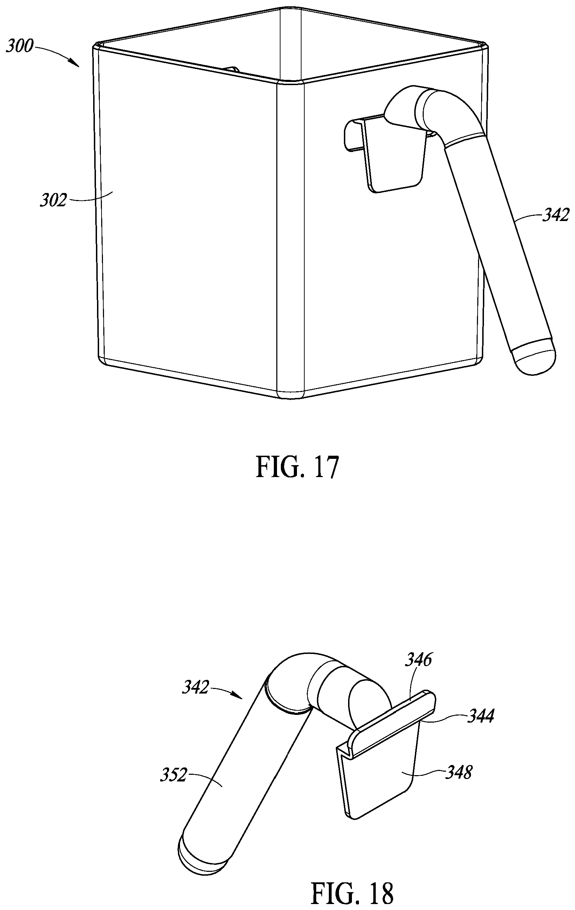

Referring to Figures 17 and 18, according to at least one implementation, one or more of the apertures 340 may be sized and positioned to receive a handle 342 that is removably coupleable to the housing 302. The handle 342 may be used to manipulate the storage cassette 300, as the housing 302 may be too cold to touch directly due to storage in the cryogenic environment and/or the presence of coolant (*e.g.,* cryogenic fluid for instance liquid nitrogen) within the interior compartment 316.

According to at least one implementation, the handle 342 may include a head portion 344 that engages the storage cassette 300. As shown, the head portion 344 may include a first flange 346 insertable through one of the apertures 340 (*e.g.,* the second aperture 340b) to releasably couple the handle 342 to the storage cassette 300. The head portion 344 may include a second flange 348 that abuts the outer facing surface 314 of the at least one sidewall 306. The handle 342 may further include a grip 352 extending from the head portion 344, the grip 352 being sized to be held in a hand of an operator to facilitate manipulation of the storage cassette 300 via movement of the handle 342.

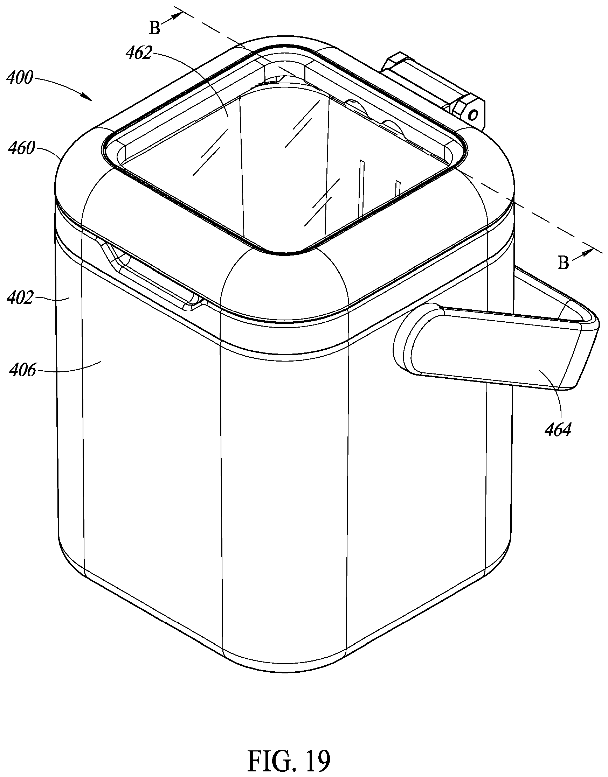

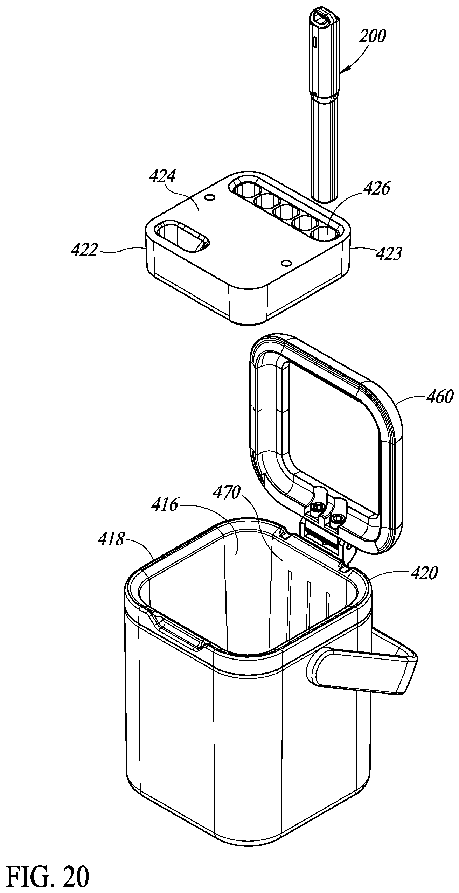

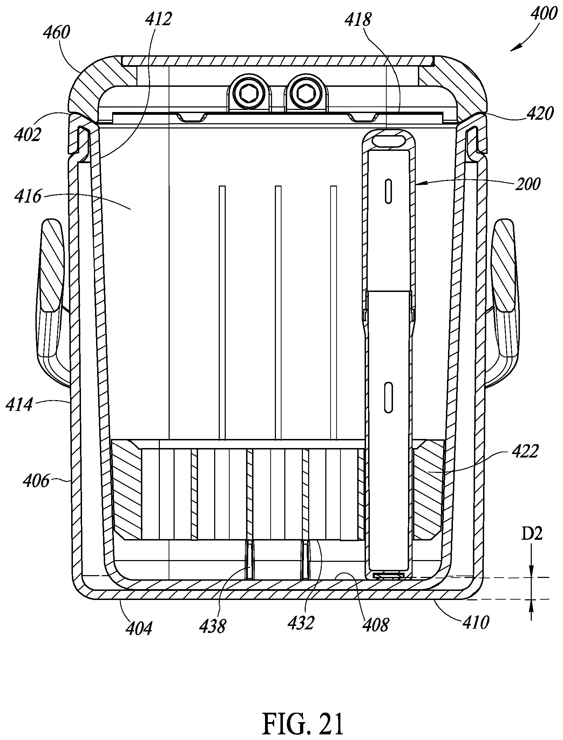

Referring to Figures 1 and 19 to 21, the cryogenic storage system 100 may include a second bulk carrier, for example a specimen transporter 400. The specimen transporter 400 is similar to the storage cassette 300, as described in reference to Figures 14 to 16, such that portions of the description of the storage cassette 300 are applicable to the specimen transporter 400, with differences called out below.

Rather than carrying a plurality of the specimen containers 200 to be stored in the cryogenic freezer 102 as described above in relation to the storage cassettes 300, the specimen transporter 400 may hold one or more of the specimen containers 200 as they are transported to or from the cryogenic freezer 102, (*e.g.,* after harvesting or prior to implantation). The one or more of the specimen containers 200 may be carried by the specimen transporter 400, for example arrayed in a two-dimensional array (*e.g.,* 7x7, 10x10, 8x12, and 14x14).

The specimen transporter 400 maintains cryogenic conditions for an array of the specimen containers 200, for example up to 49 separate ones of the specimen containers 200, according to at least one implementation. As shown in the illustrated embodiment, the specimen transporter 400 may include a housing 402 having a bottom 404 and at least one sidewall 406. The bottom 404 may have an inner facing surface 408 and an outer facing surface 410. The at least one sidewall 406 may have an inner facing surface 412 and an outer facing surface 414. According to at least one implementation, the housing 402 of the specimen transporter 400 includes a double wall, such that the inner facing surface 408 and the outer facing surface 410 may be part of separate walls separated by a gap (*e.g.,* an air gap, a vacuum gap, an insulation gap). Similarly, the inner facing surface 412 and the outer facing surface 414 may be part of separate walls separated by a gap.

The inner facing surface 408 of the bottom 404 and the inner facing surface 412 of the at least one sidewall 406 may delineate an interior compartment 416 of the housing 402. The interior compartment 416 may have an interior compartment profile, and the housing 402 may have an opening 418 at a top 420 thereof, formed by the top 420, which is opposite the bottom 404 with respect to a vertical dimension, as shown in the illustrated embodiment.

The specimen transporter 400 may further include a tray 422 that supports one or more (*e.g.,* a plurality of) the specimen containers 200 within the interior compartment 416 of the housing 402. The tray 422 may include an outer perimeter 423 that corresponds to the inner facing surface 412 of the at least one sidewall 406 such that the tray 422 is insertable into the interior compartment 416, and once inserted relative movement of the housing 402 and the tray 422 is inhibited. According to at least one implementation, the storage cassette 300 includes a seated configuration in which the tray 422 abuts the housing 402 such that further vertical movement of the tray 422 towards the bottom 404 of the housing 402 is prevented, and lateral movement of the tray 422 relative to the housing 402 is also prevented. In the seated configuration, vertical movement of the tray 422 away from the bottom 404, and towards the opening 418 at the top 420 of the housing 402 may not be inhibited, thereby allowing removal of the tray 422 from the interior compartment 416.

According to at least one implementation, the tray 422 may be the same as the tray 322, such that the same tray 322 is able to be inserted into both the interior compartment 316 and the interior compartment 416 to carry an array of the specimen containers 200. According to another implementation, the tray 422 may function similarly to the tray 322 as described above, but may have a different size, shape, or both such that the tray 422 fits within the interior compartment 416 of the specimen transporter 400, but does not fit inside the interior compartment 316 of the storage cassette 300. The tray 422 may include the plurality of substrates as described above in reference to the tray 322. Alternatively, the tray 422 may include a single substrate 424 with one or more (*e.g.,* a plurality of) through holes 426. The number of the plurality of through holes 426 may match, or may be different than the number of the plurality of through holes 326 of the storage cassette 300.

The housing 402 may include a number of set offs 438 that extend upwardly from the inner facing surface 408 of the bottom 404 into the interior compartment 416. As shown in the illustrated implementation, the set offs 438 may be positioned to support the tray 422, for example by abutting a bottom surface 432 of the tray 422 such that the tray 422 is spaced from the inner facing surface 408 of the bottom 404 of the housing 402. The spacing of the tray 422 from the inner facing surface 408 delineates a volume in which coolant may collect. As shown, the tray 422 may support the specimen container 200 such that the distal portion 212 of the specimen container 200 extends into the volume and is at least partially surrounded by the coolant (*e.g.,* cryogenic fluid), if present.

The specimen transporter 400 may include a thickness of: the bottom 404, the tray 422, or both, such that a portion of the specimen container 200, for example the wireless transponder 208, is within a defined distance D2 of the outer facing surface 410 of the bottom 404. According to one embodiment, specimen container 200 is supported directly by (*i.e.,* is in direct contact with) the inner facing surface 408, such that the defined distance D2 is equal to a thickness of the bottom 404 of the housing 402 plus a thickness of the bottom of the specimen container 200. According to at least one implementation, the distance D1 and the distance D2 are equal.

The specimen transporter 400 may include a thickness of the tray 422, the set offs 438, or both, such that a specimen 220 within the specimen container 200, is surrounded by the tray 422 (in the lateral direction) and the tray 422 is spaced upward and away from the wireless transponder 208.

As shown, the specimen transporter 400 may include a cover 460 (e.g., in the form of a lid) to seal the opening 418. The at least one sidewall 406 may be devoid of any apertures, for example the first, second, and third apertures 327, 329, and 331, and the bottom 404 may be devoid of any apertures, such that when the cover 460 seals the opening 418, the interior compartment 416 is completely sealed from the external environment.

Thus, the interior compartment 416 of the specimen transporter 400 may be filled with an amount of coolant and then sealed to maintain an acceptable temperature for any specimen containers 200 contained therein. The specimen transporter 400 may include a double layer wall to ensure thermal stability of the interior compartment 416 when sealed from the external environment. The cover 460 may be permanently, for example pivotally, attached to the housing 402. The cover 460 may include a transparent portion 462 that provides visibility to the interior compartment 416 when the cover 460 is closed and sealing the opening 418.

The specimen transporter 400 may include a handle 464 that facilitates lifting the specimen transporter 400, for example by a human hand(s). The handle 464 may take the form of a rigid member. As shown in the illustrated implementation, the rigid member may be a U-shaped member. The handle 464 may be rotatably attached to the housing 402, for example to laterally opposite surfaces of the at least one sidewall 406, as shown.

The handle 464 may be rotatable into an "up" position to facilitate lifting and carrying of the specimen transporter 400. According to at least one implementation, when in the "up" position there may be enough clearance between the handle 464 and the cover 460 to allow passage of fingers of a user of the specimen transporter 400. According to another implementation, when the handle is in the "up" position fully opening the cover 460 may be blocked by interference with the handle 464. The handle 464 may be rotatable into a "down" position in which the handle 464 does not interfere with movement of the cover 460 (*i.e.,* during transition from a closed configuration to an open configuration).

The handle 464 may take other forms. For example the handle 464 may be fixed relative to the housing 402 and the cover 460. The handle 464 may be in the form of a depression, or textured surface, for example in the at least one sidewall 406. According to one embodiment, the handle 464 may be a non-rigid member (*e.g.,* a belt, a strap, etc.).

The specimen transporter 400 may include a vertically oriented thermal shunt 470 positioned within the interior compartment 416. The vertically oriented thermal shunt 470 may include a material with a high thermal capacity (ability to store thermal energy). Materials with a higher thermal capacity require more energy for a given temperature change than a material with a lower thermal capacity. Thermal capacity for a given material can be calculated by multiplying the volume times the density times the specific heat times the temperature change. When comparing the thermal capacity of two different materials, if the volume and the temperature change for the two materials is the same, the densities and the specific heats of the two materials determines the relative thermal capacities of the two materials.

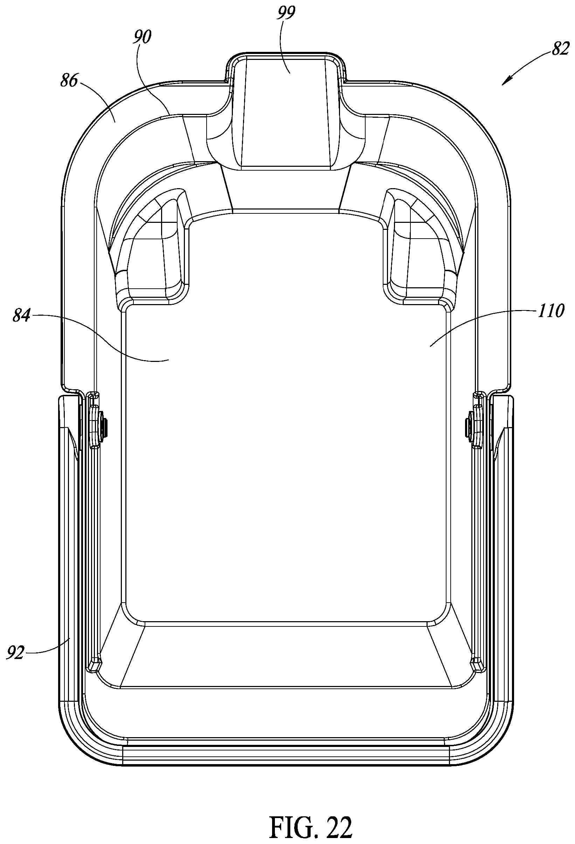

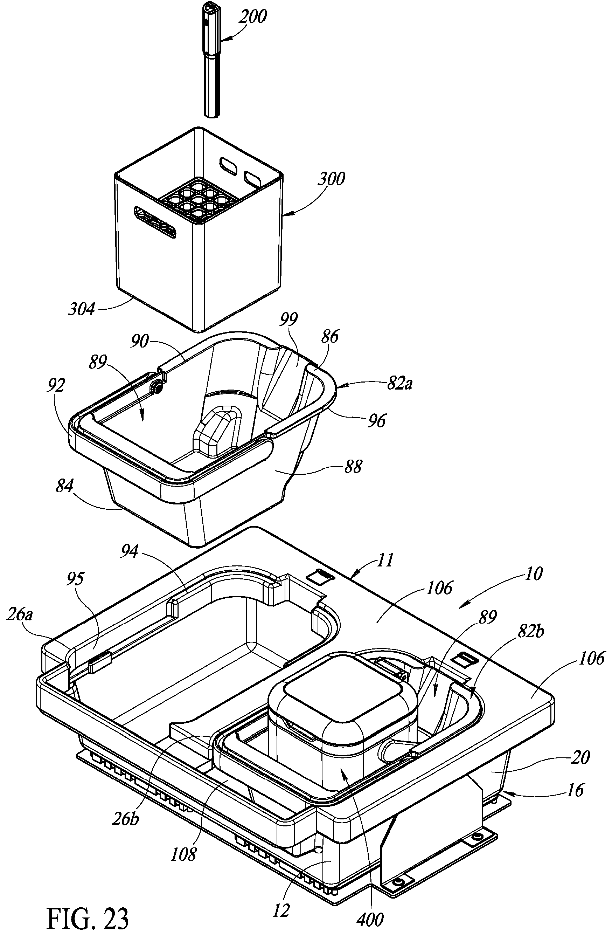

Referring to Figures 22 to 24, the cryogenic storage system 100 may include at least one bucket 82 that may hold a volume of coolant (*e.g.,* liquid nitrogen) and may support at least one bulk carrier (*e.g.,* the storage cassette 300 or the specimen transporter 400) such that the at least one of the storage cassette 300 and the specimen transporter 400 is at least partially submerged within the volume of coolant.

As shown, the at least one bucket 82 may include a first bucket 82a and a second bucket 82b. Alternatively, the cryogenic system 100 may include one larger bucket 82 that includes the features and provides the functions of both of the separate first and second buckets 82a, 82b as described below. The first bucket 82a includes a bottom 84, a top 86, at least one sidewall 88 extending from the bottom 84 to the top 86 with an opening 90 at the top 86, forming a reservoir 89 which may, or may not, hold cryogenic fluid (*e.g.,* nitrogen in liquid and/or vapor forms). The opening 90 may be sized to receive at least partially therein a container, such as one of the bulk carriers (*e.g.,* the storage cassette 300, the specimen transporter 400, etc.) that carries at least one specimen container (*e.g.,* the specimen container 200), therein.

The first bucket 82a may include a handle 92 coupled to the least one sidewall 88. The handle 92 may be pivotally coupled such that the handle 92 pivots between a deployed position and an un-deployed position. Alternatively, the handle 92 may be fixed, for example monolithic with, the first bucket 82a. The at least one bucket 82 may include the first bucket 82a and a second bucket 82b. The first bucket 82a and the second bucket 82b may be identical, and may be able to receive both the storage cassette 300 and the specimen transporter 400 (one at a time). Alternatively, the first bucket 82a and the second bucket 82b may have different structures, such that the first bucket 82a includes features that correspond to the storage cassette 300 and the second bucket 82b includes features that correspond to the specimen transporter 400.

The frame 11 and/or well 12 may support both the first bucket 82a and the second bucket 82b simultaneously, such that both the first bucket 82a and the second bucket 82b are removably, receivable by the at least one opening 26 in the frame 11 and into the open top 22 of the well 12. As shown, the first bucket 82a may be removably, receivable by the first opening 26a in the frame 11 and open top 22 of the well 12 and the second bucket 82b may be removably, receivable by the second opening 26b in the frame 11 and open top 22 of the well 12.

According to at least one implementation, the frame 11 and the at least one bucket 82 include complementary structures that engage to properly vertically space and laterally align the at least one bucket 82 with the frame 11 and the well 12, and hence with the antenna array 38a, 38b (Figure 8). The complementary structures may be shaped such that when the complementary structures are engaged, relative movement of the at least one bucket 82 with respect to the frame 11 and the well 12 is inhibited (*e.g.,* prevented, restrained) in one or more directions and degrees of freedom. According to at least one implementation, movement of the at least one bucket 82 relative to the frame 11 and the well 12 is blocked in all directions except for vertically upward and away from the frame 11 and the well 12, which is uninhibited to facilitate removal of the at least one bucket 82 from the opening 26.

The complementary structures may include a recess 94 that extends into the frame 11 and is shaped to match at least a portion of a shape of a shoulder 96 of the at least one bucket 82. As shown, when in a seated configuration a top surface 98 of the first bucket 82a may be flush with a top surface 106 of the frame 11. The recess 94 may be sized to accommodate the handle 92 so that the handle 92 is also flush with the top surface 106 of the frame 11.

According to at least one implementation, the first bucket 82a may be held by the handle 92 (*e.g.,* in the vertical orientation) while the first bucket 82a is positioned within the first opening 26a. After the first bucket 82a is in the seated configuration (*i.e.,* the shoulder 96 is fully seated within the recess 94) the handle 92 may be rotated to fit within a portion 95 of the recess 94 such that the handle 92 is flush with the top surface 106 of the frame 11. The recess 94 may include a cutout 108 positioned so as to be adjacent the handle 92 when the handle 92 is stowed flush with the top surface 106, so as to assist with rotation of the handle 92 back to the vertical orientation to facilitate removal of the first bucket 82a from the well 12.

The at least one bucket 82 may include a spout 99. The spout 99 may be part of the complementary structure, for example the recess 94 may be shaped to correspond to and at least partially capture the spout 99.

The at least one bucket 82 and the storage cassette 300 may include complementary structures that engage to properly vertically space and laterally align the storage cassette 300 and the at least one bucket 82, and hence with the antenna array 38a, 38b (Figure 8). The complementary structures of the at least one bucket 82 and the storage cassette 300 may be shaped such that when they are engaged relative movement of the storage cassette 300 and the at least one bucket 82 is inhibited (*e.g.,* prevented) in one or more directions and degrees of freedom. According to at least one implementation, movement of the storage cassette 300 relative to the at least one bucket 82 is blocked in all directions except for vertically upward and away from the at least one bucket 82, which is uninhibited to facilitate removal of the storage cassette 300 from the at least one bucket 82. The complementary structures may include a recess 110 (*e.g.,* that extends into or is at least partially delineated by the bottom 84) of the at least one bucket 82 and the bottom 304 of the storage cassette 300.

Similarly, the at least one bucket 82 and the specimen transporter 400 may include complementary structures that engage to properly vertically space and laterally align the specimen transporter 400 relative to the at least one bucket 82. The complementary structures of the at least one bucket 82 and the specimen transporter 400 may be shaped such that when they are engaged relative movement of the specimen transporter 400 and the at least one bucket 82 is inhibited (*e.g.,* prevented) in one or more directions and degrees of freedom.

According to one embodiment, movement of the specimen transporter 400 relative to the at least one bucket 82 is blocked in all directions except for vertically upward and away from the at least one bucket 82, which is uninhibited to facilitate removal of the specimen transporter 400 from the at least one bucket 82. The complementary structures may include the recess 110 and the bottom 404 of the specimen transporter 400, which may be the same shape as the bottom 304 of the storage cassette 300. Alternatively, the complementary structures may include a recess shaped differently than the recess 110 and the bottom 404 of the specimen transporter 400 may be a different shape than the shape of the bottom 304 of the storage cassette 300.

When the complementary structures of the frame 11 and/or well 12 and one of the at least one bucket 82 (*e.g.,* the first bucket 82a) are engaged, the complementary structures of the first bucket 82a and the storage cassette 300 are engaged, each of the through holes 326 is aligned with a respective one of the antennas 34. According to at least one implementation, each of the through holes 326 is laterally aligned with a respective one of the antennas 34 along one or two lateral dimensions such that the beam axis 58 of the respective antenna 34 intersects the through hole 326 (*e.g.,* is aligned with a central axis of the through hole 326). Thus, when aligned as described above, the beam axis 58 of the antenna 34 intersects the wireless transponder 208 of the specimen containers 200 positioned within the through hole 326. According to at least one implementation, each of the antennas 34 in the array 38 is vertically spaced a nominal distance (within a defined tolerance) from any specimen containers 200 (and their associated wireless transponder 208) positioned in the through holes 326 .

When the complementary structures of the frame 11 and/or well housing 12 are engaged with the structures of one of the at least one bucket 82 (*e.g.,* the second bucket 82b), the complementary structures of the second bucket 82b and the specimen transporter 400 are engaged, each of the through holes 426 is vertically spaced from an laterally aligned with a respective one of the antennas 34. According to at least one implementation, each of the through holes 426 is laterally aligned with a respective one of the antennas 34 such that the beam axis 58 of the respective antenna 34 intersects the through hole 426 (*e.g.,* is aligned with a central axis of the through hole 426). Thus, when aligned as described above, the beam axis 58 of the antenna 34 intersects the wireless transponder 208 of the specimen containers 200 positioned within the through hole 426. According to at least one implementation, each of the antennas 34 in the array 38 is laterally aligned with respective through holes 426 and any specimen containers 200 (and their associated wireless transponder 208) positioned therein, and is also vertically spaced from the specimen containers 200 (and their associated wireless transponder 208) by a nominal distance (*e.g.,* 5 mm, 8 mm, 10 mm, 17 mm, 20 mm) within a defined tolerance (*e.g., +*/*-10%).*

As shown, the at least one array 38 of antennas 34 may be positioned so as to be within a defined or nominal vertical distance D3 of a portion, for example the wireless transponder 208, of the specimen container 200 when one of the storage cassette 300 or the specimen transporter 400 carrying the specimen container 200 is positioned in the at least one bucket 82, and the at least one bucket 82 is in the seated configuration with respect to the frame 11 and well 12. As shown in the illustrated embodiment, the vertical or nominal distance D3 may be measured from the antenna 38, through the insulator 54, through the bottom 18 of the well 12, through the bottom 84 of the at least one bucket 82, and through the bottom 304, 404 of either the storage cassette 300 or the specimen transporter 400 to the wireless transponder 208 of the specimen container 200. The vertical or nominal distance D3 may additionally include any gaps therebetween (*e.g.,* an air gap 112 between the bottom 18 of the well 12 and the bottom 84 of the at least one bucket 82). According to at least one implementation, the vertical or nominal distance D3 is less than 25 mm, for example less than 20 mm, for example about 17 mm. To prevent interference, each of the structures through which the vertical distance D3 is measured may be formed from one or more materials that are transparent to wavelengths employed in RFID interrogation (*e.g.,* radio frequencies, microwave frequencies).

Proper spacing and alignment of the antennas 34 and the wireless transponders 208 enables accurate identification of individual ones of the wireless transponders 208, and thereby identification of the associated specimen container 200, and thereby identification of the specimen carried by the specimen container 200, for example via comparison of received signal strength (*e.g.,* RSSI). This accurate identification enables a number of the specimen containers 200 to be tightly packed, for example in a 7x7 array, to limit the volume that needs to be kept at a cryogenic temperature to maintain the viability of the specimen.

Referring to Figures 1 to 24, the cryogenic storage system 100 may be used to perform a method of cryogenically storing one or more biological specimens or samples (specimens and samples are used interchangeably herein). The method may include collecting one or more biological specimen from one or more entities. Each biological specimen is positioned within the vial 204 of one of the specimen containers 200. Each of the specimen containers 200 carrying a biological specimen is placed within the specimen transporter 400. According to one embodiment, each of the specimen containers 200 is positioned within a through hole 426 of the tray 422.

The interior compartment 416 of the specimen transporter 400 may maintain a cryogenic environment. According to one embodiment, a volume of coolant *(e.g.,* liquid nitrogen) is contained within the interior compartment 416 to at least partially surround the specimen container 200. Additional specimen containers 200 may be placed within the interior compartment 416 until all of the through holes 426 of the tray 422 are occupied by one of the specimen containers 200. The cover 460 may be closed thereby sealing the interior compartment 416 from the surrounding, ambient environment.

The specimen transporter 400 may then be moved to a location with the cryogenic freezer 102 where the specimen containers 200 can be stored and maintained at cryogenic conditions long-term. The specimen transporter 400 may be placed within one of the buckets 82, for example the second bucket 82b. Prior to placement of the specimen transporter 400 within the reservoir 89 of the bucket 82, the bucket 82 may be placed in the seated configuration with respect to the frame 11 and well 12. A volume of coolant (*e.g.,* cryogenic fluid, for instance nitrogen in liquid and/or vapor form) may be added to the reservoir 89 of the second bucket 82b such that when the specimen transporter 400 is positioned within the reservoir 89 of the second bucket 82b, at least a portion of the specimen transporter 400 is submerged within the coolant.

The specimen transporter 400 may be aligned with the second bucket 82b, for example by engaging the recess 110 and the bottom 404 of the specimen transporter 400. Once the recess 110 and the bottom 404 are engaged, the through holes 426 and the wireless transponders 208 within each of the specimen containers 200 positioned within the through holes 426 are vertically spaced from an laterally aligned with a respective one of the antennas 34 of the second array 38b. Optionally, a wireless transponder (not shown) of the specimen transporter 400 may be aligned with an antenna 34 of the second printed circuit board 32b.

One of the storage cassettes 300 may be placed within the reservoir 89 of one of the buckets 82, for example the first bucket 82a. The storage cassette 300 may be retrieved from within the cryogenic freezer 102, and may be supporting one of more specimen containers 200. Alternatively, the storage cassette 300 may be empty prior to placement within the reservoir 89 of the first bucket 82a. Prior to placement of the storage cassette 300 within the reservoir 89 of the bucket 82, the bucket 82 may be placed in the seated configuration with respect to the bucket housing 12. A volume of coolant (*e.g.,* cryogenic fluid, for instance nitrogen in liquid and/or vapor form) may be added to the reservoir 89 of the first bucket 82a such that when the storage cassette 300 is positioned within the reservoir 89 of the first bucket 82a, at least a portion of the storage cassette 300 is submerged within the coolant.

The storage cassette 300 may be aligned with the first bucket 82a, for example by engaging the recess 110 and the bottom 304 of the storage cassette 300. Once the recess 110 and the bottom 304 are engaged, the through holes 326 and the wireless transponders 208 of the specimen containers 200 positioned within the through holes 326 are vertically spaced from and laterally aligned with a respective one of the antennas 34 of the first array 38a. Optionally, a wireless transponder (not shown) of the storage cassette 300 may be aligned with an antenna 34 of the first printed circuit board 32a.

One or more of the specimen containers 200 within the specimen transporter 400 may be transferred to the storage cassette 300. Prior to removal of any of the specimen containers 200, each of the wireless transponders 208 are interrogated by the RFID reader 30, thereby identifying each of the specimen containers 200 uniquely from all others of the specimen containers 200, and cataloguing the position of each of the specimen containers 200 within the specimen transporter 400.

Removal of the specimen containers 200 from the specimen transporter 400 is recognized by the RFID reader 30, and upon placement of the removed specimen containers 200 within the storage cassette 300, the chain-of-custody or database associated with the specimen container 200 is updated with the new position of the specimen container 200 within the storage cassette 300, (*e.g.,* the specific through hole 326 of the tray 322 in which the specimen container 200 is positioned). Retrieval of biological specimen from the cryogenic freezer 102 may undergo a similar method, with the movement of the specimen container 200 reversed as it is transferred from the storage cassette 300 to the specimen transporter 400.

Once transfer of the specimen containers 200 from the specimen transporter to the storage cassette 300 is complete, the storage cassette 300 may be stored in the cryogenic freezer 102 (*e.g.,* in vertical stacks, the vertical stacks also called racks). The stacks or racks of storage cassettes 300 may be annularly arrayed in the cryogenic freezer 102 about a central axis of the cryogenic storage tank or freezer 102. The cryogenic freezer 102 may include a turntable or conveyer in the interior thereof, on which the stacks or racks of storage cassettes 300 are carried. This allows respective stacks or racks of storage cassettes 300 to be aligned with an opening of the cryogenic freezer 102 for placement or removal.

Referring again to Figure 1, the transfer station 104 may include a transfer surface 114 having a planar portion 116 and a peripheral wall 118 that extends upwardly from the planar portion 116 about at least a portion of a periphery 120 of the transfer surface 114. A portion of the peripheral wall 118, for example at the back 122 of the transfer station 104, may include an angled face 124 that slopes downward from the back 122 of the transfer station 104 towards the front 126 of the transfer station 104. As shown in the illustrated embodiment, the transfer surface 114 may provide a path from the cryogenic freezer 102 to the interrogation station 10. For example, the storage cassette 300 may exit the cryogenic storage freezer 102, then slide down the angled face 124, across the planar portion 116 to interrogation station 10.

Referring to Figures 1 to 24, at least one of the arrays 38 of the antennas 34 may be operable to read information from one or more of the wireless transponders 208 physically associated with respective specimen containers 200, storage cassettes 300, and/or specimen transporters 400. As explained in detail herein, the at least one array 38 of antennas 34 may include at least one one-dimensional or two-dimensional array of antennas, and one or more transmitters, receivers, transceivers (collectively radios), operable to cause the antennas 34 to emit interrogation signals and to receive response signals in response to the interrogations signals.

The transfer station 104 may include a dedicated user interface system including one or more of: one or more display screens, one or more touch-sensitive display screens, one or more speakers, one or more microphones, one or more keyboards, one or more pointer devices (*e.g.,* computer mouse, trackpad, trackball), one or more haptic interfaces. The user interfaces are communicatively coupled *(e.g.,* wired, optical, wireless or radio) with processor(s) via one or more peripheral interfaces to provide user input to the processor(s) and to receive output from the processor(s) to be presented to a user. In particular, the processor(s) may execute processor-executable instructions that cause the processor(s) to cause devices to present a user interface (*e.g.,* a graphical user interface), for instance via a touch screen display.

Portions of the cryogenic storage system 100 may be of a conventional design. For example, the cryogenic freezer 102 and/or the picker or elevator 140 that removes storage cassettes 300 from the cryogenic freezer 102 may take the form of a commercially available automated storage system (*e.g.,* the Bistore III Cryo -190°C System sold by Brooks Life Sciences).

In at least some implementations, the cryogenic storage system 100 may be designed to operate with existing lab equipment, for example with conventional specimen containers (*e.g.,* FluidX^{™} 24-Format tubes, FluidX^{™} 48-Format tubes FluidX^{™} 96- Format tubes available from Brooks Life Sciences) or with plates or racks (*e.g.,* 5 ¼ inch by 5 ¼ inch plates or wells, Vision Plate^{™} 96 Well plate, Vision Plate^{™} 24 Well plate, 10x10 Cryo Rack rack, 14x14 Cryo Rack rack available from Brooks Life Sciences). Designing the cryogenic storage system 100 to operate with existing lab equipment places a number of constraints on the structure as well as the operation. For example, a two-dimensional array of antennas may be designed to successfully interrogate RFID transponders arrayed in the various available formats. This can place limits on the size (*e.g.,* coil size) of the antennas used to interrogate and received response signals from RFID transponders (*e.g.,* passive RFID transponders or "tags").

Smaller antennas typically reduce the range, which may require higher transmit power to compensate for the loss of range. This may be particularly true where the antennas of the wireless transponders are subject to extremely cold temperatures, for instance when the wireless transponders are immersed in a liquid nitrogen bath, while antennas of a reader are at room temperature. Also, relatively close spacing dictated by conventional equipment formats can increase the amount of cross-talk during interrogation, where an interrogation signal from one antenna excites and elicits response signals from more than one wireless transponder. Cross-talk may also increase with increasing transmit power, for instance where antenna size must be small, for instance to accommodate a spacing dictated by conventional spatial formats. The user of existing wireless transponders and readers (*e.g.,* RFID interrogators or RFID readers) can also require specific accommodations to be made in structure and/or operation. For example, RFID readers typically employ automatic gain control.

The cryogenic storage system 100 may comprise: the plurality of antennas 34 spatially arrayed in a two-dimensional array 38, the two-dimensional array 38 having a set of dimensions or orders (*e.g.,* 2x2, 3x3, 5x5, 4x6, 7x7, 9x9, 8x12). One or more radios may be communicatively coupled to drive the antennas 34 to emit interrogation signals to interrogate the wireless transponders 208 and to receive response signals from any of the wireless transponders 208 in a range of one or more of the antennas 34. There may, for example, be a single radio which is multiplexed to the various antennas 34. Alternatively, there may be one radio per antenna 34. Alternatively, the antennas 34 may be grouped into sets, for instance five sets, and one radio per set, multiplexed to the antennas 34 of the respective sets. In some instances the radios are transmitters, in some the radios are receivers, and in yet other instances the radios are transceivers.

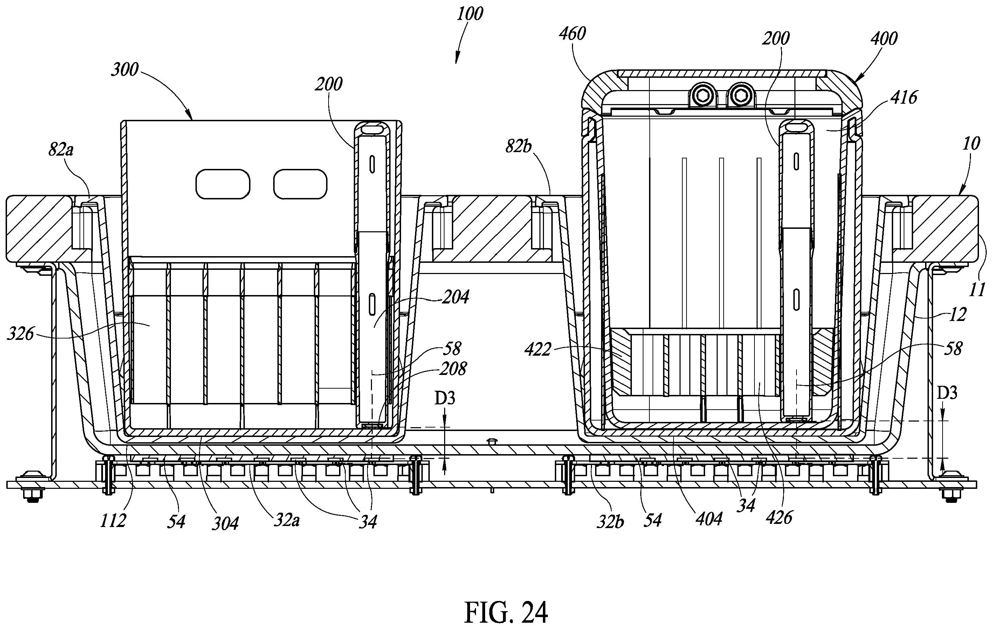

Referring to Figures 24 and 25, a processor-based transfer system 1122 of, or that interfaces with, the cryogenic storage system 100 of Figure 1 may facilitate transfers, whether automated or manual, of the specimen containers 200 between the storage cassettes 300 and the specimen transporters 400. The storage cassettes 300 may be designed for long term storage in cryogenic freezers (*e.g.,* tanks or dewars), which are typically large and heavy fixtures. The specimen transporters 400 may be designed for temporary storage, in a format that is portable.

Additional examples of suitable storage cassettes, and of the specimen transporters, which can temporarily maintain cryogenic materials at cryogenic temperatures, as wells as the specimen containers for use therewith, are described in U.S. patent application 62/900,281, filed September 13, 2019; U.S. patent application 62/880,786, filed July 31, 2019; U.S. patent application 62/879,160, filed July 26, 2019; U.S. patent application 62/741,986, filed October 5, 2018; and U.S. patent application 62/741,998, filed October 5, 2018.

The processor-based transfer system 1122 may include one or more RFID reader integrated circuits 1400a, 1400b, 1400c, 1400d, 1400e, 1400f, 1400g, 1400h, 1400i, 1400j (ten shown, collectively 1400), one or more arrays of antennas 1401a, 1401b (two arrays of antennas shown, collectively 1402), a control subsystem 1402, and a user interface system 1404. The processor-based transfer system 1122 optionally includes one or more robots, for example one or more co-robots 1405 (only one shown) that interface with the picker or elevator 140 (Figure 1) of the cryogenic storage system 100.

The RFID reader integrated circuits 1400a-1400j wirelessly interrogate and read information stored in the wireless transponders 208 physically associated with the specimen containers 200 and/or the storage cassettes 300 and/or the specimen transporters 400. The RFID reader integrated circuits 1400a-1400d each include one more transmitters, receivers or transceivers, collectively and individually referred to herein as radios. The RFID reader integrated circuits 1400a-1400j are each communicatively coupled to one or more antennas of the antenna arrays 1401a, 1401b), and are operable to emit wireless signals (*e.g.,* interrogation signals) and/or to receive wireless signals (*e.g.,* response signals) returned from the wireless transponders 208. The wireless signals are typically in the radio or microwave frequency bands of the electromagnetic spectrum.

Also for example, one or more machine-readable symbol readers or scanners 1460 may optically read information stored or encoded in one or more machine-readable symbols (*e.g.,* one-dimensional or barcode symbols, two-dimensional or area code symbols) carried by, printed on, or inscribed in the specimen containers 200, the storage cassettes 300, and the specimen transporters 400.

The antenna(s) 1401a, 1401b may include antennas 34 from the at least one array 38, for example, formed or carried on one side of the printed circuit board 32, while various electrical or electronic components (*e.g.,* inductors, resistors, capacitors, RFID reader integrated circuits 1400) may be carried on the other side of the printed circuit board 32, thereby providing a substantially flat planner surface that faces the specimen containers during use, and minimizing a distance therebetween *(e.g.,* less than or equal to 2 mm, less than 10 mm, less than 17 mm, less than 20 mm).

The control subsystem 1402 may include one or more processors 1406, for example, one or more of: one or more microcontrollers, one or more microprocessors, one or more central processing units, one or more digital signal processors (DSPs), one or more graphics processing units (GPUs), one or more application specific integrated circuits (ASICs), one or more field programmable gate arrays (FPGAs), and/or one or more programmable logic controllers (PLCs). The control subsystem 1402 may include one or more nontransitory storage media, for example, one or more nonvolatile storage media and/or one or more volatile storage media, for example a system memory 1408 that includes one or more of: one or more read only memories (ROMs) 1410, one or more random access memories (RAMs) 1412, one or more FLASH memory, one or more magnetic disk 1414 and associated drives 1416, one or more optical disk drives 1418 and associated drives 1420, one or more solid state drives 1422, one or more cache memories, and/or one or more registers of one or more processors 1406.

The control subsystem 1402 may include one or more communications channels 1424 (*e.g.,* buses) that communicatively couple the processor(s) with the storage media. The control subsystem 1402 may include one or more communications ports, for example one or more wired communications ports 1426, wireless communications ports 1428 (*e.g.,* Wi-Fi and/or Bluetooth radios and associated antennas 1430; infrared transceivers) that provide for communications between the control subsystem 1402 and external devices (*e.g.,* dedicated control system of conventional cryogenic refrigerator system with, or without picker or elevator 140).

The processor(s) 1406 of the control subsystem 1402 are operable to execute logic, for example to execute one or more algorithms stored as process-executable instructions by the one or more nontransitory storage media. Suitable algorithms are set out herein. Process-executable instructions may, for example, include a basic input/output operating system (BIOS) 1432, for example stored in ROM 1410. Process-executable instructions may, for example, include an operating system (OS) 1434, for example stored in RAM 1412 during execution. Process-executable instructions may, for example, include one or more application programs 1436, which provide the logic to collect user information, map transfers between storage and carrier cassettes, verify that the specimen containers are at the correct positions in the carrier or storage cassettes, and establish evidence of a chain-of-custody for the same, the applications program(s) stored, for example, in RAM 1412 during operation.

Process-executable instructions may include one or more other programs or modules 1438, for example to provide for communications with external devices and/or to control operation of co-robot(s) 1405, and which may be stored, for example, in RAM 1412 during execution. One or more data structures 1440 may store information, for example information that identifies specific users, identifies specific clinicians, identifies specific patients, identifies specific procedures, identifies specific specimen containers and associates the specific specimen containers with specific patients, and that maps specimen containers to respective storage cassettes and/or carrier cassettes. The data structures 1440 may take a variety of forms including databases, data sets, records and fields, tables, linked lists, trees, binary trees, etc. The data structures 1440 may be stored, for example, in RAM 1412 during execution.

The processor(s) 1406 of the control subsystem 1402 are communicatively coupled operable (*e.g.,* wired, optical, wireless or radio) to receive information from the RFID reader integrated circuits 1400a-1400j, and optionally to control operation of one or more of the RFID reader integrated circuits 1400a-1400j. The processor(s) 1406 of the control subsystem 1402 are also operable to receive user input from, and provide user out to, one or more user interface devices of the user interface system 1404, to allow a human user to interact with the processor-based transfer system 1122.

The user interface system 1404 may, for example, include one or more of: one or more display screens (*e.g.,* two display screens), one or more touch-sensitive display screens 1442, one or more speakers 1444, one or more microphones 1446, one or more keyboards 1448, one or more pointer devices 1450 (*e.g.,* computer mouse, trackpad, trackball), one or more haptic interfaces. The user interfaces 1404 are communicatively coupled (*e.g.,* wired, optical, wireless or radio) with the processor(s) via one or more peripheral interfaces 1452a, 1452b to provide user input to the processor(s) 1406 and to receive output from the processor(s) 1406 to be presented to a user. In particular, the processor(s) 1406 may execute processor-executable instructions that cause the processor(s) to cause devices to present a user interface (*e.g.,* a graphical user interface), for instance via a touch screen display 1442. Various user interface elements are illustrated and described herein.

In some instances the radios are transmitters, in some the radios are receivers, and in yet other instances the radios are transceivers. The processor-based control system 1122 may be communicatively coupled to the radio(s). The processor-based control system 1122 determines, based on response signals received in response to a given interrogation, whether at least one of the wireless transponders 208 is located at an expected position of one of the storage cassette 300 or the specimen transporter 400. In response to a determination that the at least one of the wireless transponders 208 is not located at the expected position of one of the storage cassette 300 or the specimen transporter 400 the processor-based control system 1122 may cause a signal indicative of an occurrence of an unexpected condition to be provided. To cause a signal indicative of an occurrence of an unexpected condition to be provided, the processor-based control system 1122 may cause a visual prompt to be presented and/or cause a signal to be provided to a robot that causes the robot to make a movement.

For each response signal received in response to a given interrogation, the processor-based control system 1122 may determine which response signal was returned from one of the wireless transponders 208 that is closest wireless transponder 208 to the antenna 34 that emitted the respective interrogation signal to which the wireless transponders 208 are responding. For example, for each response signal received in response to a given interrogation, at least one of the processor-based control system 1122 or the at least one radio may normalize a respective received signal strength indicator (RSSI) value that indicates a received signal strength of the response signal to account for any automatic gain adjustment introduced by the at least one radio.

For instance, for each interrogation, the processor-based control system 1122 determines which of the respective normalized RSSI values for the response signals received in response to a given interrogation has the largest absolute value. To determine which of the respective normalized RSSI values for the response signals received in response to a given interrogation has the largest absolute value for each interrogation, the processor-based control system 1122 may compare the respective normalized RSSI values for the received response signals to one another.

To determine which response signal was returned from one of the wireless transponders 208 that is closest wireless transponder 208 to the antenna 34 that emitted the respective interrogation signal to which the wireless transponders 208 are responding, the processor-based control system 1122 may further compare a respective read rate to one another for each of the wireless transponders 208 that respond, the respective read rate representative of a total number of times the respective wireless transponder 208 is read per a unit of time. To determine which response signal was returned from one of the wireless transponders 208 that is the closest wireless transponder 208 to the antenna 34 that emitted the respective interrogation signal to which the wireless transponders 208 are responding, the processor-based control system may additionally or alternatively further compare a respective response time to one another for each of the wireless transponders 208 that respond, the respective response time representative of an amount of time the respective wireless transponder 208 takes to initially respond to the interrogation.

Before the processor-based control system 1122 determines whether at least one of the wireless transponders 208 is located at an expected position of one of the storage cassette 300 or the specimen transporter 400, the processor-based control system 1122 may further cause a prompt to be presented which indicates a position of one of the storage cassette 300 or the specimen transporter 400 from which to transfer one of the specimen containers 200 along with the wireless transponder 208 associated therewith and a position of the other of the storage cassette 300 or the specimen transporter 400 to which to transfer the specimen container 200, the expected position being the position of the other of the storage cassette 300 or the specimen transporter 400 to which the specimen container 200 is to be transferred.

The processor-based control system 1122 may further determine, based on response signals received in response to one or more interrogations, whether there are any specimen containers 200 marked by respective ones of the wireless transponders 208 in an unexpected position in one of the storage cassette 300 or the specimen transporter 400, and in response to a determination that there is at least one specimen container 200 marked by respective one of the wireless transponders 208 in an unexpected position in one of the storage cassette 300 or the specimen transporter 400, cause a signal indicative of an occurrence of an unexpected condition to be provided.

The processor-based control system 1122 may further determine, based on response signals received in response to one or more interrogations, whether there are any specimen containers 200 marked by respective ones of the wireless transponders 208 that are missing from the one of the storage cassette 300 or the specimen transporter 400, and in response to a determination that there is at least one specimen containers 200 marked that is missing from the one of the storage cassette 300 or the specimen transporter 400, cause a signal indicative of an occurrence of an unexpected condition to be provided. To cause a signal indicative of an occurrence of an unexpected condition to be provided, the processor-based control system 1122 may cause a presentation of a signal to be provided that is indicative of the occurrence of the unexpected condition and indicative of at least one of an incorrect location and a correct location for the specimen container 200 in the at least one of the storage cassette 300 or the specimen transporter 400.

Additionally, speed is typically important to commercial operation. To increase speed, antennas may be grouped into a plurality of sets, the sets which are operated in parallel with one another. For example, a two-dimensional array of ninety-six antennas, arranged as a 8 x 12 array, may be grouped to four sets denominated A, B, C D, and corresponding to four quadrants of the two-dimensional array (*e.g.,* sets A, B, C D arranged clockwise starting with A in an upper left quadrant). The position in each quadrant may be represented by a pair of integers representing a row and column. In operation, a first antenna 1,1 of a first set A, a first antenna 1,1 of a second set B, a first antenna 1,1 of a third set C, and a first antenna 1,1 of a fourth set D, may be operated to concurrently transmit respective interrogation signals (*e.g.,* carrier waves) during a first interrogation cycle.

The other antennas of the first set A, second set B, third set C, and fourth set D may be monitored for response signals as part of the first interrogation cycle. Subsequently, a second antenna 1,2 of the first set A, a second antenna 1,2 of the second set B, the second antenna 1,2 of a third set C, and the second antenna 1,2 of the fourth set D, may be operated to concurrently transmit respective interrogation signals (*e.g.,* carrier waves) during a second interrogation cycle. The other antennas of the first set A, second set B, third set C, and fourth set D may be monitored for response signals as part of the second interrogation cycle.

Subsequent interrogation cycles may cycle through the remaining antennas of the sets, operating one of the antennas from each set to transmit interrogations currently with one another. In some implementations, operation may result in there being a fixed distance between the transmitting antennas of any two sets. For instance, during any given interrogation cycle the transmitting antenna in one set may be in the same relative position within its own set as the relative position of the other transmitting antennas in the other sets. This is similar to the operation described above, where the row/column pair of the transmitting antenna of each set match one another during each interrogation cycle.

Alternatively, operation may result in there being a maximum achievable distance between the concurrently transmitting antennas during any given interrogation cycle. For instance, a first antenna 1,1 of a first set A, a sixth antenna 1,6 of a second set B, a nineteenth antenna 4,1 of a third set C, and a twenty-fourth antenna 4,6 of a fourth set D, may be operated to concurrently transmit respective interrogation signals (*e.g.,* carrier waves) in a first interrogation cycle. Thus, during the first interrogation cycle antennas at the opposite four corners of the two-dimensional array concurrently transmit interrogation signals. During subsequent interrogation cycles, the antennas in each set A, B, C, D may be stepped through in an order that matches one another, for example transmitting interrogation signals from antennas successively along a row, then successively from a next row, etc.

While four sets are described, any number of sets of antennas may be employed. While two patterns of antenna activation are described, other patterns may be employed. Also, while generally described as employing all antennas to monitor for a response signal except the antenna that most recently emitted the interrogation signal, other approaches may be employed. For example, all antennas may be monitored, including the antenna that most recently emitted the interrogation signal. In such implementations, delay will typically be employed to allow resonance in the transmitting antenna to decay sufficiently as to allow detection of a response signal. Alternatively, only a select one or select ones of the antennas may be employed to monitor for response signals. For instance, in some implementation, only the antenna that most recently transmitted the interrogation signal is monitored for a receipt of a response signal, typically after a delay time.

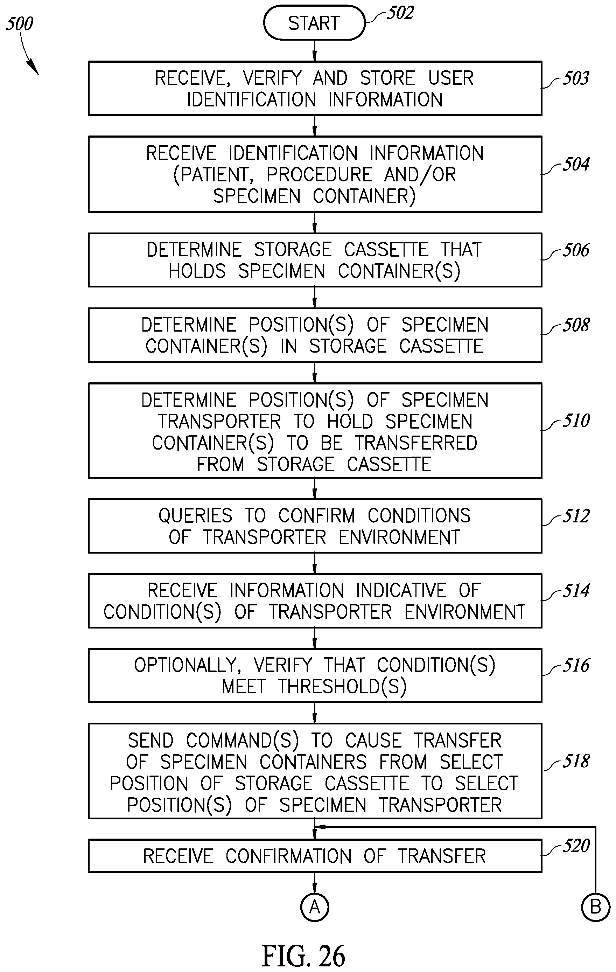

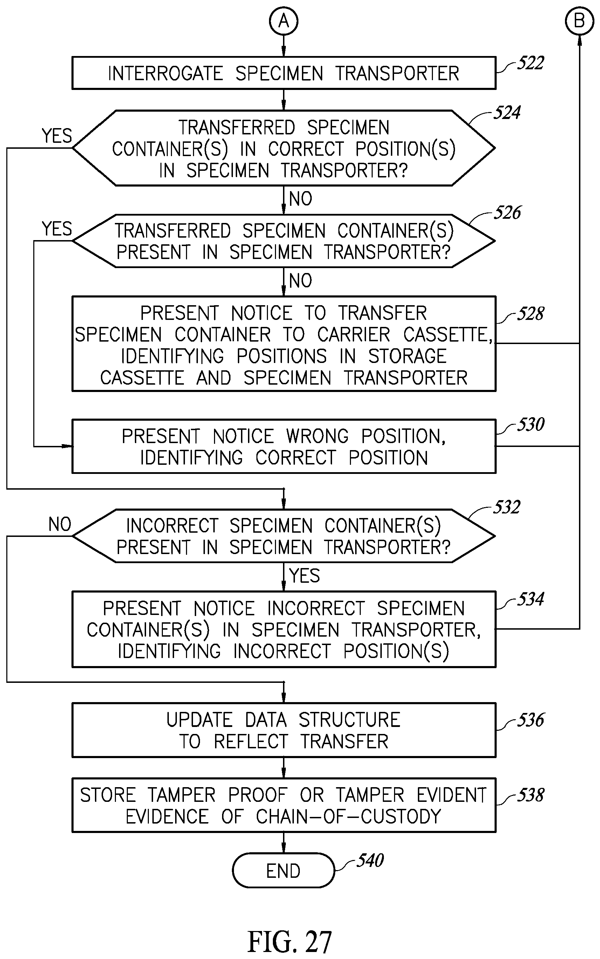

Referring to Figures 24 to 27 a method 500 of operation to transfer specimen containers (*e.g.,* the specimen containers 200) from storage cassettes (*e.g.,* the storage cassettes 300), which can be stored in a cryogenic freezer (*e.g.,* the cryogenic freezer 102), to a portable thermally insulated cryogenic carrier (*e.g.,* the specimen transporter 400), is described according to at least one illustrated implementation. The method 500 may be performed when the storage cassette 300 and the specimen transporter 400 are each positioned within the interrogation station 10 such that the specimen containers 200 contained therein are aligned with the antennas 34 as described above.

The method 500 starts at 502. For example, the method may start in response to a powering on of the processor-based transfer system 1122 or a component thereof, in response to detection of the storage cassette 300 and/or the specimen transporter 400 at a reader, receipt of a user input, or a call from a calling routine or program.

At 503, the processor-based transfer system 1122 receives, verifies and stores user identification information. The user identification information may take a variety of forms, for example a user identifier, a password or passphrase, and/or biometric data (*e.g.,* digital fingerprint, digital iris scan, facial features).

At 504, the processor-based transfer system 1122 receives identification information. The identification information may, for example, be received as user input via a user interface, or as an electronic transfer of information from another system, for instance from a clinical procedure management processor-based system. The identification information may, for example, include a patient name, unique patient identifier, patient data of birth, procedure type or unique procedure identifier, specimen container identifier, and/or specimen identifier.

At 506, the processor-based transfer system 1122 determines which of the storage cassettes 300 that holds the one or more specimen containers 200 to be retrieved. For example, one or more of the specimen containers 200 may hold biological specimens or tissue to be used in a given procedure on a given patient. The processor-based transfer system 1122 may, for example, query a data structure using the identification information.

At 508, the processor-based transfer system 1122 determines one or more positions of the identified specimen containers 200 in the storage cassette 300. The processor-based transfer system may, for example, query a data structure using the identification information.

At 510, the processor-based transfer system 1122 determines position(s) of the specimen transporter 400 to hold the specimen container(s) 200 transferred from the storage cassette 300. The processor-based transfer system 1122 may, for example, query a data structure using the identification information.

At 512, the processor-based transfer system 1122 causes a presentation of queries to confirm conditions of carrier environment. Queries may be presented visually or aurally via a user interface. Queries may include a request that the user confirm that the specimen transporter 400 is ready, that there is adequate coolant (*e.g.,* liquid nitrogen) in the specimen transporter 400, and that the coolant is adequately cold (*e.g.,* at or below approximate negative 190° C).

At 514, the processor-based transfer system 1122 receives information indicative of condition(s) of an environment of the specimen transporter 400. The information may, for example, be received via a user interface.

Optionally at 516, the processor-based transfer system 1122 verifies that one or more conditions meet one or more thresholds. For example, a user may enter a fluid level and/or a temperature of the fluid, which can be compared to a threshold level and/or threshold temperature.

At 518, the processor-based transfer system 1122 sends one or more commands to cause transfer of the specimen containers 200 from select position of the storage cassette 300 to select position(s) of the specimen transporter 400. In the case of manual transfers, the commands may be presented to a user as prompts via the user interface. In the case of automated transfers, the commands can be in the form of a motion plan for execution by a robot or other mechanical conveyance.

At 520, the processor-based transfer system 1122 receives a confirmation of transfer. Confirmation may be received from a user via a user interface. Alternatively, confirmation may come from a processor-based system, for example a robot, indicating that a series of operations have been completed.

At 522, the processor-based transfer system 1122 interrogates the specimen transporter 400. For example, a reader may sequentially interrogate each position in the specimen transporter 400, determining which positions have a wireless transponder tagged specimen container, and even the identity of each specimen container at each position that has a specimen container. Such may, for example, be implemented via an RFID reader or interrogator (*e.g.,* the RFID reader 30), with a two-dimensional array of antennas (*e.g.,* the antennas 34) which are arranged to be in registration with respective positions of the specimen transporter 400 and spaced at a nominal distance therefrom.

At 524, the processor-based transfer system 1122 determines whether the transferred specimen container(s) 200 are in correct the position(s) in the specimen transporter 400. The processor-based transfer system 1122 can query a data structure to determine, for each position of the specimen transporter 400 that is supposed to have a specimen container 200, the identity of the specimen container 200 that is supposed to be at that position. The processor-based transfer system 1122 compares the actual mapping, as determined via interrogation, with the intended mapping, to verify that each specimen container 200 is in the correct position, to identify specimen containers 200 in incorrect positions, to identify missing specimen containers 200 and/or specimen containers 200 that should not be in the specimen transporter 400, as described below.

If it is determined at 524 that the transferred specimen container(s) 200 are not in correct position(s) in the specimen transporter 400, then the processor-based transfer system 1122 attempts to determine what specific errors have occurred. For example, at 526 the processor-based transfer system 1122 determines whether the transferred specimen container(s) 200 are present in the specimen transporter 400. If it is determined at 526 that the transferred specimen container(s) 200 are not present in the specimen transporter 400, then at 528 the processor-based transfer system 1122 causes a user interface to present a prompt to transfer the specimen container 200 from the storage cassette 300 to the specimen transporter 400, identifying the specific positions in the storage cassette 300 and the specimen transporter 400, and control returns to 520 to await receipt of a confirmation that the transfer has been completed.

Alternatively, the processor-based transfer system 1122 provides instructions to a robot to implement the transfer of the specimen container(s) 200. If it is determined at 526 that the transferred specimen container(s) 200 is (are) present in the specimen transporter 400, then at 530 the processor-based system 1122 causes the user interface to present a notification or prompt to move the specimen container(s) 200 to the correct position(s) in the specimen transporter 400, including an identification of the position from which the specimen container 200 should be moved from and the position to which the specimen container 200 should be moved. Control then returns to await receipt of a confirmation that the transfer has been completed at 520.

If it is determined at 524 that the transferred specimen container(s) 200 are in correct position(s) in the specimen transporter 400, then optionally at 532 the processor-based transfer system 1122 determines whether there are any incorrect specimen container(s) 200 present in the specimen transporter 400. If it is determined at 532 that there are incorrect specimen container(s) 200 present in the specimen transporter 400, then at 534 the processor-based transfer system 1122 causes the user interface to present a notification that there are incorrect specimen container(s) 200 in the specimen transporter 400, identifying incorrect position(s). Control then returns to await receipt of a confirmation that the transfer has been completed at 520. If it is determined at 532 that there are not incorrect specimen container(s) 200 present in the specimen transporter 400, then control passes to 536.

At 536, the processor-based transfer system 1122 updates a data structure to reflect transfer. The data structure can, for example take the form of a database, table, tree structure or linked list.

At 538, the processor-based transfer system 1122 stores evidence of chain-of-custody, preferably in a tamper proof or tamper evident form. The evidence of chain-of-title may, for example be stored in a block-chain form.

The method 500 terminates at 540, for example until invoked again.

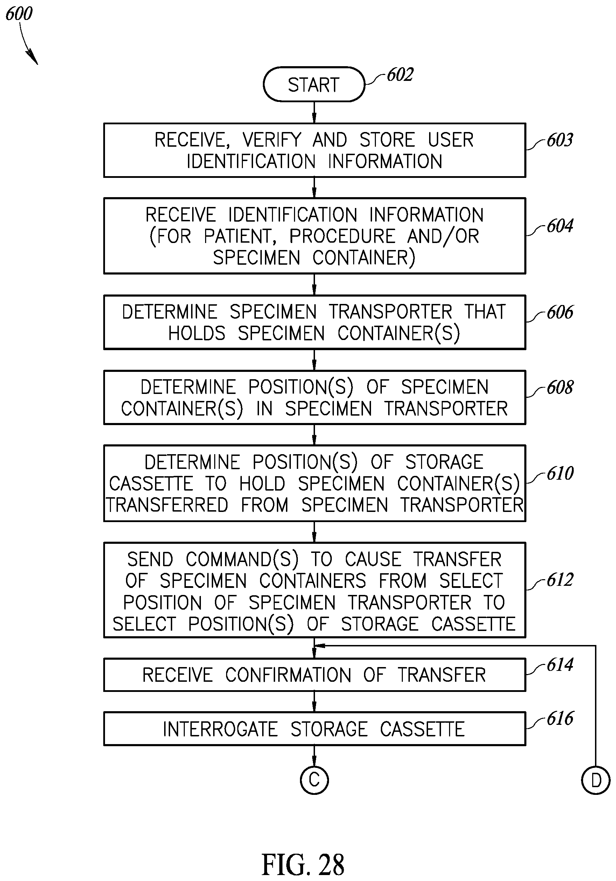

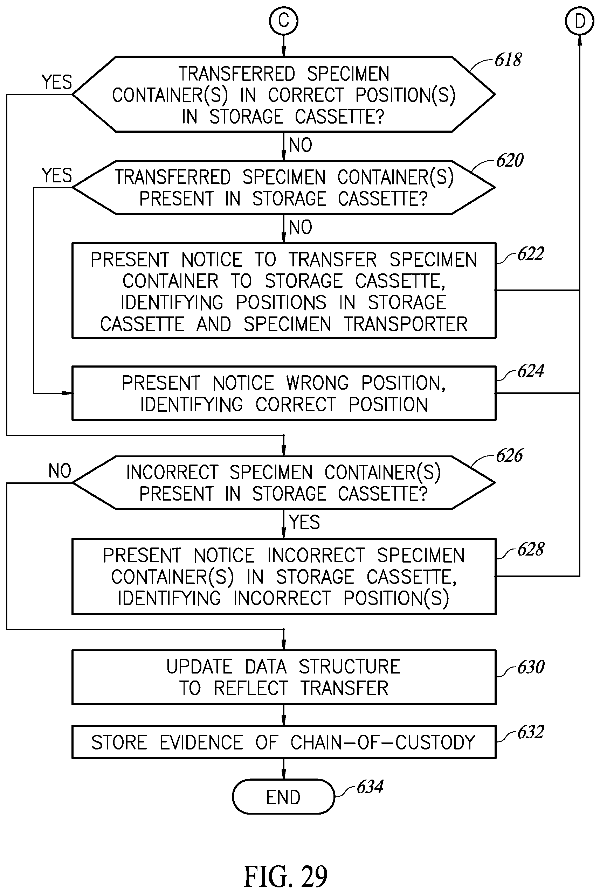

Referring to Figures 24 to 25 and 28 to 29 a method 600 of operation to transfer specimen containers (*e.g.,* the specimen containers 200) from storage cassettes (*e.g.,* the storage cassettes 300), which can be stored in a cryogenic freezer (*e.g.,* the cryogenic freezer 102), to a portable thermally insulated cryogenic carrier (*e.g.,* the specimen transporter 400), is described according to at least one illustrated implementation. The method 500 may be performed when the storage cassette 300 and the specimen transporter 400 are each positioned within the interrogation station 10 such that the specimen containers 200 contained therein are aligned with the antennas 34 as described above.

The method 600 starts at 602. For example, the method may start in response to a powering on of the processor-based transfer system 1122 or a component thereof, in response to detection of the storage cassette 300 and the specimen transporter 400 at a reader, receipt of a user input, or a call from a calling routine or program.

At 603, the processor-based transfer system 1122 receives, verifies and stores user identification information. The user identification information may take a variety of forms, for example a user identifier, a password or passphrase, and/or biometric data (*e.g.,* digital fingerprint, digital iris scan, facial features).

At 604, the processor-based transfer system 1122 receives identification information. The identification information may, for example, be received as user input via a user interface, or as an electronic transfer of information from another system, for instance from a clinical procedure management processor-based system. The identification information may, for example, include a patient name, unique patient identifier, patient data of birth, procedure type or unique procedure identifier, specimen container identifier, and/or specimen identifier.

At 606, the processor-based transfer system 1122 determines which specimen transporter 400 holds the one or more specimen containers 200 to be stored in the cryogenic storage freezer 102. For example, one or more specimen containers 200 may hold biological specimens or tissue collected from a given procedure on a given patient. The processor-based transfer system 1122 may, for example, query a data structure using the identification information.

At 608, the processor-based transfer system 1122 determines one or more positions of the identified specimen containers in the specimen transporter 400. The processor-based transfer system 1122 may, for example, query a data structure using the identification information.

At 610, the processor-based transfer system 1122 determines position(s) of storage cassette to hold specimen container(s) transferred from the specimen transporter 400. The processor-based transfer system 1122 may, for example, query a data structure using the identification information.

At 612, the processor-based transfer system 1122 sends one or more commands to cause transfer of specimen containers from select position of the specimen transporter 400 to select position(s) of the storage cassette 300. In the case of manual transfers, the commands may be presented to a user as prompts via the user interface. In the case of automated transfers, the commands can be in the form of a motion plan for execution by a robot or other mechanical conveyance.

At 614, the processor-based transfer system 1122 receives a confirmation of transfer. Confirmation may be received from a user via a user interface. Alternatively, confirmation may come from a processor-based system, for example a robot, indicating that a series of operations have been completed.

Optionally at 616, the processor-based transfer system 1122 interrogates the storage cassette 300. For example, a reader may sequentially interrogate each position in the storage cassette 300, determining which positions have a wireless transponder tagged specimen container 200, and even the identity of each specimen container at each position that has a specimen container 200. Such may, for example, be implemented via an RFID reader or interrogator, with a two-dimensional array of antennas 34 which are arranged to be in registration with respective positions of the storage cassette 300 and spaced vertically at a nominal distance with respect thereto.

Optionally at 618, the processor-based transfer system 1122 determines whether the transferred specimen container(s) 200 are in correct the position(s) in the storage cassette 300. The processor-based transfer system 1122 can query a data structure to determine, for each position of the storage cassette 300 that is supposed to have a specimen container 200, the identity of the specimen container 200 that is supposed to be at that position. The processor-based transfer system 1122 compares the actual mapping, as determined via interrogation, with the intended mapping, to verify that each specimen container 200 is in the correct position, to identify specimen containers 200 in incorrect positions, to identify missing specimen containers 200 and/or specimen containers 200 that should not be in the storage cassette 300, as described below.

If it is determined at 618 that the transferred specimen container(s) 200 are not in correct position(s) in storage cassette 300, then at 620 the processor-based transfer system 1122 determines whether the transferred specimen container(s) 200 are present in storage cassette 300. If it is determined at 620 that the transferred specimen container(s) 200 are not present in storage cassette 300, then at 622 the processor-based transfer system 1122 causes a user interface to present a prompt to transfer the specimen container 200 from the specimen transporter 400to the storage cassette 300, identifying the specific positions in the specimen transporter 400 and the storage cassettes 300. Alternatively, the processor-based transfer system 1122 provides instructions to a robot to implement the transfer of the specimen container(s) 200. Control then returns to await receipt of a confirmation that the transfer has been completed at 614. If it is determined at 620 that the transferred specimen container(s) 200 is (are) present in the storage cassette 300, then at 624 the processor-based system 122 causes the user interface to present a notification or prompt to move the specimen container(s) 200 to the correct position(s) in the storage cassette 300, including an identification of the position from which the specimen container 200 should be moved from and the position to which the specimen container 200 should be moved. Control then returns to await receipt of a confirmation that the transfer has been completed at 614.

If it is determined at 618 that the transferred specimen container(s) 200 are in correct position(s) in the storage cassette 300, then optionally at 626 the processor-based transfer system 1122 determines whether there are incorrect specimen container(s) 200 present in the storage cassette 300. If it is determined at 620 that there are incorrect specimen container(s) 200 present in the storage cassette 300, then at 628 the processor-based transfer system 1122 causes the user interface to present a notification that there are incorrect specimen container(s) 200 in the storage cassette 300, identifying incorrect position(s). Control then returns to await receipt of a confirmation that the transfer has been completed at 614. If it is determined at 620 that there are not incorrect specimen container(s) 200 present in the storage cassette 300, then control passes to 630.

At 630, the processor-based transfer system 1122 updates a data structure to reflect transfer. The data structure can, for example take the form of a database, table, tree structure or linked list.

At 632, the processor-based transfer system 1122 stores evidence of chain-of-custody, preferably in a tamper proof or tamper evident form. The evidence of chain-of-title may, for example be stored in a block-chain form.

The method 600 terminates at 634, for example until invoked again.

The invention is defined in the claims, and the various implementations and embodiments described above can be combined to provide further implementations and embodiments. In this respect, further prior art is given in all of the commonly assigned U.S. patent application publications, U.S. patent applications, foreign patents, and foreign patent applications referred to in this specification and/or listed in the Application Data Sheet, including but not limited U.S. patent application 62/900,281, filed September 13, 2019; U.S. patent application 62/880,786, filed July 31, 2019; U.S. patent application 62/879,160, filed July 26, 2019; U.S. patent application 62/741,986, filed October 5, 2018; U.S. patent application 62/741,998, filed October 5, 2018; U.S. patent application 62/927,566, filed October 29, 2019; U.S. patent application 63/082,789, filed September 24, 2020; U.S. patent application 63/128,732, filed December 21, 2020; and U.S. patent application 63/186,593, filed May 10, 2021.

In general, in the following claims, the terms used should not be construed to limit the claims to the specific implementations and embodiments disclosed in the specification and the claims, but should be construed to include all possible implementations and embodiments along with the scope of the claims.

## Claims

1. An interrogation station (10) comprising:
a frame (11) and a well (12), the well (12) having a bottom (18), a top (22) opposite the bottom (18), and at least one sidewall (20) that extends upwardly from the bottom (18) towards the top (22), wherein the bottom (18), the at least one sidewall (20), and the top (22) collectively delineate an interior cavity (24) of the well (12), at least a portion of the top (22) of the well (12) being open, the frame (11) including at least one opening (26) that provides passage through the open top (22) of the well (12) so as to provide access to the interior cavity (24) from an exterior thereof;
at least one array (38) of antennas (34) arranged within a plane (P1); and
a coupler (56) that secures the at least one array (38) of antennas (34) beneath the bottom (18) of the well (12) at a defined perpendicular distance from the bottom (18) of the well (12) in a vertical direction, and with the at least one array (38) of antennas (34) laterally aligned with a respective one of the at least one opening (26) of the frame (11);
the interrogation station **characterized by** at least one printed circuit board (32) which carries the at least one array (38) of antennas (34),
wherein the coupler (56) includes a slotted plate (60) which supports the at least one printed circuit board (32) at a location between the well (12) and the slotted plate (60), and the slotted plate (60) includes a plurality of slots that extend through a thickness of the slotted plate (60) and that are aligned with components mounted on a lower side (62) of the at least one printed circuit board (32) along the vertical direction, and optionally comprising a hydrophobic material (74) positioned between at least a portion of the slotted plate (60) and a portion of the at least one printed circuit board (32), said at least a portion of the slotted plate (60) in particular including multiple, discrete portions of the slotted plate (60), and/or being devoid of any slots that extend through the thickness of the slotted plate (60).

2. The interrogation station (10) of claim 1 wherein
the at least one opening (26) of the frame (11) includes a first opening (26a) and
a second opening (26b) separated from one another by a portion of the frame (11) that is positioned between the first opening (26a) and the second opening (26b) along a lateral direction that is perpendicular to the vertical direction;
the at least one array (38) of antennas (34) optionally:
a) including a first array of antennas and a second array of antennas, and the first array (38a) of antennas (34) is laterally aligned with the first opening (26a) along a first lateral direction and a second lateral direction, and the second array (38b) of antennas (34) is aligned with the second opening (26b) along the first lateral direction and the second lateral direction, the first array (38a) of antennas (34) in particular including a plurality of rows of antennas (34), each of the plurality of rows being evenly spaced from adjacent ones of the plurality of rows along the first lateral direction that is perpendicular to the vertical direction, and a plurality of columns of antennas (34), each of the plurality of columns being evenly spaced from adjacent ones of the plurality of columns along the second lateral direction that is perpendicular to both the vertical direction and the first lateral direction; and/or
b) positioned beneath the bottom (18) of the well (12) at a location in which the at least one array (38) of antennas (34) is less than 5 mm from the bottom (18) of the well (12) as measured along the vertical direction; and/or
c) positioned such that an insulator (54) optionally comprised by the interrogation station (10) of is positioned between the bottom (18) of the well (12) and the at least one array (38) of antennas (34).

3. The interrogation station (10) of claim 1, wherein the coupler (56) includes a pair of brackets (76) that secure the slotted plate (60) to the well (12), in particular wherein the coupler (56) secures the printed circuit board (32) to the well (12) without entirely enclosing the printed circuit board (32), and the interrogation station (10) comprise an integral unit sized to be mounted in an opening at least adjacent a surface (114) of a transfer station (104).

4. The interrogation station (10) of claim 1, further comprising:
at least one bucket (82) including a bucket bottom (84) and at least one bucket sidewall (88) extending from the bucket bottom (84) such that the bucket bottom (84) and the at least one bucket sidewall (88) collectively delineate a reservoir (89) to contain a volume of coolant, wherein the at least one bucket (82) is receivable in the at least one opening (26) of the frame (11) such that the bucket bottom (84) is laterally aligned with the at least one array (38) of antennas (34) along the first and the second lateral directions.

5. The interrogation station (10) of claim 4, wherein the at least one bucket (82) and the frame (11) include complementary structures shaped such that when the complementary structures are engaged movement of the at least one bucket (82) relative to the frame (11) and the well (12) is blocked in all directions except for upward and away from the well (12) along the vertical direction, in particular such that
when the complementary structures are engaged and movement of the at least one bucket (82) relative to the frame (11) and the well (12) is blocked in all directions except for upward and away from the well (12) along the vertical direction: the bucket bottom (84) is separated from the bottom (18) of the well (12) by an air gap (112) and/or an entirety of the at least one bucket (82) is flush with or recessed with respect to a top surface (106) of the frame (11).

6. The interrogation station (10) of claim 5, wherein the complementary structures include a recess (94) formed by the frame (11) and at least a portion of an outer perimeter (323) of the at least one bucket (82),
said at least one bucket (82) in particular including
a) a handle (92) pivotally attached to the at least one bucket sidewall (88), and the recess (94) includes a portion to receive the handle (92), and/or
b) a first bucket (82a) and a second bucket (82b), said first bucket (82a) being identical to said second bucket (82b).

7. The interrogation station (10) of any one of claims 4 to 6, further comprising:
a bulk carrier (300) including a bulk carrier bottom (304) and at least one bulk carrier sidewall (306) that cooperatively delineate a bulk carrier interior compartment (316), the bulk carrier (300) including a tray (322) having a plurality of through holes (326) extending therethrough, each of the plurality of through holes (326) sized to receive a specimen container (200) that holds a biological specimen, wherein the bulk carrier (300) is receivable within the reservoir (89) of the at least one bucket (82) such that the plurality of through holes (326) are each laterally aligned with respective antennas (34) of the at least one array (38) of antennas (34) along the first and the second lateral directions.

8. The interrogation station (10) of claim 7, wherein the bulk carrier (300) is a first bulk carrier (300), the interrogation station (10) further comprising a second bulk carrier (300), the second bulk carrier (300) including a second tray (322) having a second plurality of through holes (326b) extending therethrough, each of the second plurality of through holes (326b) sized to receive a specimen container (200) that holds a biological specimen, wherein the second bulk carrier (300) is receivable within the reservoir (89) of the at least one bucket (82) such that the second plurality of through holes (326b) are each laterally aligned with respective antennas (34) of the at least one array (38) of antennas (34) along the first and the second lateral directions,
said first bulk carrier (300) in particular being receivable within one of the at least one bucket (82) such that the plurality of through holes (326) of the first bulk carrier (300) are each laterally aligned with a first subset of the at least one array (38) of antennas (34) along the first and the second lateral directions, and the second bulk carrier (300) is simultaneously receivable within another of the at least one bucket (82) such that the second plurality of through holes (326b) are each laterally aligned with a second subset of the at least one array (38) of antennas (34) along the first and the second lateral directions, and none of the antennas (34) of the first subset are included in the second subset, and optionally
wherein the first bulk carrier (300) and the at least one bucket (82) include complementary structures shaped such that when the complementary structures of the first bulk carrier (300) and the at least one bucket (82) are engaged, movement of the first bulk carrier (300) relative to the at least one bucket (82) is blocked in all directions except for along the vertical direction, upward and away from the at least one bucket (82), in particular wherein said complementary structures of the first bulk carrier (300) and the at least one bucket (82) include a recess (94) within the reservoir (89) of the at least one bucket (82) and a perimeter (323) of at least a portion of the first bulk carrier (82), in particular wherein the recess (94) within the reservoir (89) of the at least one bucket (82) also corresponds to at least a portion of a perimeter (323) of the second bulk carrier (300), and optionally
wherein one of the first bulk carrier (300) and the second bulk carrier (300) includes a lid (460) that is movable to close and seal the respective bulk carrier interior compartment (416), and the other of the first bulk carrier (300) and the second bulk carrier (300) is open-topped such that the respective bulk carrier interior compartment (416) open and unsealed.

9. The interrogation station (10) of claim 5, further comprising:
at least one specimen container (200) with a body that delineates an interior to at least partially enclose a biological specimen, the at least one specimen container (200) further including a wireless transponder physically associated with the body, the wireless transponder (208) carrying identification information of for the biological specimen,
wherein the body is receivable within the plurality of through holes (326) of the bulk carrier (300) such that when the bulk carrier (300) is received within the reservoir (89) of the at least one bucket (82) and the plurality of through holes (326) are each laterally aligned with respective antennas (34) of the at least one array (38) of antennas (34) along the first and the second lateral directions, the wireless transponder (208) is laterally aligned with one of the antennas (34) of the at least one array (38) of antennas (34) along the first and the second lateral directions.

10. A method of transferring biological specimen from a cryogenic freezer to a specimen transporter, the method comprising:
securing a first bucket within an internal cavity of a well (12) via a frame such that movement of the first bucket relative to the frame and well is blocked in one or more directions by interference of complementary structures of the first bucket and at least one of the frame and well;
securing a bulk carrier within a reservoir of the first bucket such that movement of the bulk carrier relative to the first bucket is blocked in one or more directions;
securing the bulk carrier relative to a first plurality of antennas (34) arranged in a first plane that is positioned beneath the well, such that movement of the bulk carrier relative to the first plurality of antennas is blocked in one or more directions and respective ones of a first plurality of through holes (326a) extending through a first tray of the bulk carrier are laterally aligned with respective ones of the first plurality of antennas along at least one lateral direction, wherein the first tray is positioned within a first interior compartment delineated by a bottom of the bulk carrier and at least one sidewall of the bulk carrier;
securing the specimen transporter (400) relative to a second plurality of antennas arranged in a second plane such that movement of the specimen transporter relative to the second plurality of antennas is blocked in one or more directions and respective ones of a second plurality of through holes (326b) extending through a second tray of the specimen transporter are laterally aligned with respective ones of the second plurality of antennas along at least one lateral direction, wherein the second tray is positioned within a second interior compartment delineated by a bottom of the specimen transporter and at least one sidewall of the specimen transporter;
securing the first plurality of antennas and the second plurality of antennas to the well by a coupler, and attaching the coupler to the well at a location above the bottom of the bulk carrier when the first bucket is secured within the internal cavity of the well and when the bulk carrier is secured within the reservoir of the first bucket;
removing a specimen container (200) enclosing a biological specimen from one of the first plurality of through holes, thereby moving a wireless transponder (208) physically associated with the specimen container out of alignment with the respective one of the first plurality of antennas that is aligned with the one of the first plurality of through holes;
inserting the specimen container (200) into one of the second plurality of through holes thereby moving the wireless transponder physically associated with the specimen container into alignment with the respective one of the second plurality of antennas that is aligned with the one of the second plurality of through holes;
after inserting the specimen container into the one of the second plurality of through holes, sealing the second interior compartment thereby thermally isolating the specimen container from a surrounding environment of the specimen transporter; and
after inserting the specimen container into the one of the second plurality of through holes, moving the specimen transporter relative to the second plurality of antennas, thereby moving all of the second plurality of through holes out of alignment with the second plurality of antennas.

11. The method of claim 10, further comprising:
securing a second bucket within the internal cavity of the well (12) such that movement of the second bucket relative to the well (12) is blocked in one or more directions by interference of complementary structures of the second bucket and the well;
wherein securing the specimen transporter relative to the second plurality of antennas includes securing the specimen transporter within a reservoir of the second bucket such that movement of the specimen transporter relative to the second bucket is blocked in one or more directions,
and wherein the second plurality of antennas is positioned beneath the well.

12. The method of claim 11, further comprising:
filling a portion of the reservoir of the first bucket with a coolant at or below -150°C; and
filling a portion of the reservoir of the second bucket with a coolant at or below -150°C.

13. The method of any one of claims 10 to 12, further comprising at least one of
a) prior to removing the specimen container from the one of the first plurality of through holes, interrogating the wireless transponder with the one of the first plurality of antennas thereby uniquely identifying the specimen container from other specimen containers positioned within the first plurality of through holes; and after inserting the specimen container into the one of the second plurality of through holes, interrogating the wireless transponder with the one of the second plurality of antennas thereby confirming that the specimen container is the same one that was removed from the one of the first plurality of through holes,
b) prior to securing the bulk carrier relative to the first plurality of antennas, removing the bulk carrier from a cryogenic environment enclosed within the cryogenic freezer,
c) maintaining the biological sample at or below -150°C for an entirety of the method,
d) securing the bulk carrier relative to the first plurality of antennas including positioning the wireless transponder that is physically associated with the specimen container within 20 mm of the respective one of the first plurality of antennas that is aligned with the one of the first plurality of through holes.

## Patentansprüche

1. Abfragestation (10) umfassend:
einen Rahmen (11) und eine Vertiefung (12), wobei die Vertiefung (12) einen Boden (18), eine dem Boden (18) gegenüberliegende Oberseite (22) und mindestens eine Seitenwand (20) aufweist, die sich vom Boden (18) nach oben in Richtung der Oberseite (22) erstreckt, wobei der Boden (18), die mindestens eine Seitenwand (20) und die Oberseite (22) gemeinsam einen Innenhohlraum (24) der Vertiefung (12) begrenzen, wobei zumindest ein Teil der Oberseite (22) der Vertiefung (12) offen ist, wobei der Rahmen (11) mindestens eine Öffnung (26) aufweist, die einen Durchgang durch die offene Oberseite (22) der Vertiefung (12) bereitstellt, um Zugang zum Innenhohlraum (24) von einer Außenseite desselben aus bereitzustellen;
mindestens eine Anordnung (38) von innerhalb einer Ebene (P1) angeordneten Antennen (34); und
einen Koppler (56), der die mindestens eine Anordnung (38) von Antennen (34) unterhalb des Bodens (18) der Vertiefung (12) in einem definierten senkrechten Abstand vom Boden (18) der Vertiefung (12) in vertikaler Richtung befestigt, und wobei die mindestens eine Anordnung (38) von Antennen (34) seitlich mit einer jeweiligen der mindestens einen Öffnung (26) des Rahmens (11) ausgerichtet ist;
wobei die Abfragestation **gekennzeichnet ist durch** mindestens eine Leiterplatte (32), die die mindestens eine Anordnung (38) von Antennen (34) trägt,
wobei der Koppler (56) eine geschlitzte Platte (60) umfasst, die die mindestens eine Leiterplatte (32) an einer Position zwischen der Vertiefung (12) und der geschlitzten Platte (60) trägt, und die geschlitzte Platte (60) eine Vielzahl von Schlitzen umfasst, die sich durch eine Dicke der geschlitzten Platte (60) erstrecken und die mit Komponenten ausgerichtet sind, die auf einer Unterseite (62) der mindestens einen Leiterplatte (32) entlang der vertikalen Richtung montiert sind, und optional ein hydrophobes Material (74) umfassend, das zwischen mindestens einem Abschnitt der geschlitzten Platte (60) und einem Abschnitt der mindestens einen Leiterplatte (32) positioniert ist, wobei der mindestens eine Abschnitt der geschlitzten Platte (60) insbesondere mehrere, diskrete Abschnitte der geschlitzten Platte (60) umfasst und/oder frei von jeglichen Schlitzen ist, die sich durch die Dicke der geschlitzten Platte (60) erstrecken.

2. Abfragestation (10) nach Anspruch 1, wobei
die mindestens eine Öffnung (26) des Rahmens (11) eine erste Öffnung (26a) und eine zweite Öffnung (26b) umfasst, die durch einen Abschnitt des Rahmens (11) voneinander getrennt sind, der zwischen der ersten Öffnung (26a) und der zweiten Öffnung (26b) entlang einer seitlichen Richtung positioniert ist, die senkrecht zur vertikalen Richtung verläuft;
wobei die mindestens eine Anordnung (38) von Antennen (34) optional:
a) eine erste Anordnung von Antennen und eine zweite Anordnung von Antennen umfasst, und die erste Anordnung (38a) von Antennen (34) seitlich mit der ersten Öffnung (26a) entlang einer ersten seitlichen Richtung und einer zweiten seitlichen Richtung ausgerichtet ist, und die zweite Anordnung (38b) von Antennen (34) mit der zweiten Öffnung (26b) entlang der ersten seitlichen Richtung und der zweiten seitlichen Richtung ausgerichtet ist, wobei die erste Anordnung (38a) von Antennen (34) insbesondere eine Vielzahl von Reihen von Antennen (34) umfasst, wobei jede der Vielzahl von Reihen gleichmäßig von benachbarten der Vielzahl von Reihen entlang der ersten seitlichen Richtung beabstandet ist, die senkrecht zur vertikalen Richtung verläuft, und eine Vielzahl von Spalten von Antennen (34), wobei jede der Vielzahl von Spalten gleichmäßig von benachbarten der Vielzahl von Spalten entlang der zweiten seitlichen Richtung beabstandet ist, die senkrecht sowohl zur vertikalen Richtung als auch zur ersten seitlichen Richtung verläuft; und/oder
b) unterhalb des Bodens (18) der Vertiefung (12) an einer Position positioniert ist, an der die mindestens eine Anordnung (38) von Antennen (34) weniger als 5 mm vom Boden (18) der Vertiefung (12) entfernt ist, gemessen entlang der vertikalen Richtung; und/oder
c) so positioniert ist, dass ein Isolator (54), der optional von der Abfragestation (10) umfasst wird, zwischen dem Boden (18) der Vertiefung (12) und der mindestens einen Anordnung (38) von Antennen (34) positioniert ist.

3. Abfragestation (10) nach Anspruch 1, wobei der Koppler (56) ein Paar von Halterungen (76) umfasst, die die geschlitzte Platte (60) an der Vertiefung (12) befestigen, insbesondere wobei der Koppler (56) die Leiterplatte (32) an der Vertiefung (12) befestigt, ohne die Leiterplatte (32) vollständig zu umschließen, und die Abfragestation (10) eine integrale Einheit umfasst, die so dimensioniert ist, dass sie in einer Öffnung zumindest angrenzend an eine Oberfläche (114) einer Transferstation (104) montiert werden kann.

4. Abfragestation (10) nach Anspruch 1, ferner umfassend:
mindestens einen Kübel (82), der einen Kübelboden (84) und mindestens eine Kübelseitenwand (88) umfasst, die sich vom Kübelboden (84) aus erstreckt, so dass der Kübelboden (84) und die mindestens eine Kübelseitenwand (88) gemeinsam eine Aufnahme (89) zur Aufnahme eines Volumens an Kühlmittel begrenzen, wobei der mindestens eine Kübel (82) in der mindestens einen Öffnung (26) des Rahmens (11) aufnehmbar ist, so dass der Kübelboden (84) seitlich mit der mindestens einen Anordnung (38) von Antennen (34) entlang der ersten und der zweiten seitlichen Richtung ausgerichtet ist.

5. Abfragestation (10) nach Anspruch 4, wobei der mindestens eine Kübel (82) und der Rahmen (11) komplementäre Strukturen umfassen, die so geformt sind, dass wenn die komplementären Strukturen ineinandergreifen eine Bewegung des mindestens einen Kübels (82) relativ zum Rahmen (11) und zur Vertiefung (12) in allen Richtungen blockiert ist, außer nach oben und weg von der Vertiefung (12) entlang der vertikalen Richtung, insbesondere so, dass
wenn die komplementären Strukturen in Eingriff stehen und die Bewegung des mindestens einen Kübels (82) relativ zum Rahmen (11) und zur Vertiefung (12) in allen Richtungen außer nach oben und weg von der Vertiefung (12) entlang der vertikalen Richtung blockiert ist: der Kübelboden (84) vom Boden (18) der Vertiefung (12) durch einen Luftspalt (112) getrennt ist und/oder der gesamte mindestens eine Kübel (82) bündig mit oder gegenüber einer Oberseite (106) des Rahmens (11) zurückgesetzt ist.

6. Abfragestation (10) nach Anspruch 5, wobei die komplementären Strukturen eine Aussparung (94) umfassen, die durch den Rahmen (11) gebildet wird, und mindestens einen Abschnitt eines Außenumfangs (323) des mindestens einen Kübels (82),
wobei der mindestens eine Kübel (82) insbesondere umfasst:
a) einen Griff (92), der schwenkbar an der mindestens einen Kübelseitenwand (88) befestigt ist, und die Aussparung (94) einen Abschnitt zur Aufnahme des Griffs (92) umfasst, und/oder
b) einen ersten Kübel (82a) und einen zweiten Kübel (82b), wobei der erste Kübel (82a) identisch mit dem zweiten Kübel (82b) ist.

7. Abfragestation (10) nach einem der Ansprüche 4 bis 6, ferner umfassend:
einen Sammelbehälter (300), der einen Sammelbehälterboden (304) und mindestens eine Sammelbehälterseitenwand (306) umfasst, die gemeinsam ein Sammelbehälter-Innenkompartiment (316) begrenzen, wobei der Sammelbehälter (300) ein Tablett (322) mit einer Vielzahl von Durchgangslöchern (326) umfasst, die sich durch dieses erstrecken, wobei jedes der Vielzahl von Durchgangslöchern (326) so dimensioniert ist, dass es einen Probenbehälter (200) aufnehmen kann, der eine biologische Probe enthält, wobei der Sammelbehälter (300) innerhalb der Aufnahme (89) des mindestens einen Kübels (82) aufnehmbar ist, so dass die Vielzahl von Durchgangslöchern (326) jeweils seitlich mit jeweiligen Antennen (34) der mindestens einen Anordnung (38) von Antennen (34) entlang der ersten und der zweiten seitlichen Richtung ausgerichtet sind.

8. Abfragestation (10) nach Anspruch 7, wobei der Sammelbehälter (300) ein erster Sammelbehälter (300) ist, wobei die Abfragestation (10) ferner einen zweiten Sammelbehälter (300) umfasst, wobei der zweite Sammelbehälter (300) ein zweites Tablett (322) mit einer zweiten Vielzahl von Durchgangslöchern (326b) umfasst, die sich durch dieses erstrecken, wobei jedes der zweiten Vielzahl von Durchgangslöchern (326b) so dimensioniert ist, dass es einen Probenbehälter (200) aufnehmen kann, der eine biologische Probe enthält, wobei der zweite Sammelbehälter (300) innerhalb der Aufnahme (89) des mindestens einen Kübels (82) aufnehmbar ist, so dass die zweite Vielzahl von Durchgangslöchern (326b) jeweils seitlich mit jeweiligen Antennen (34) der mindestens einen Anordnung (38) von Antennen (34) entlang der ersten und der zweiten seitlichen Richtung ausgerichtet sind,
wobei der erste Sammelbehälter (300) insbesondere innerhalb eines des mindestens einen Kübels (82) aufnehmbar ist, so dass die Vielzahl von Durchgangslöchern (326) des ersten Sammelbehälters (300) jeweils seitlich mit einer ersten Teilmenge der mindestens einen Anordnung (38) von Antennen (34) entlang der ersten und der zweiten seitlichen Richtung ausgerichtet ist, und der zweite Sammelbehälter (300) gleichzeitig innerhalb eines anderen des mindestens einen Kübels (82) aufnehmbar ist, so dass die zweite Vielzahl von Durchgangslöchern (326b) jeweils seitlich mit einer zweiten Teilmenge der mindestens einen Anordnung (38) von Antennen (34) entlang der ersten und der zweiten seitlichen Richtung ausgerichtet ist, und keine der Antennen (34) der ersten Teilmenge in der zweiten Teilmenge enthalten ist, und optional
wobei der erste Sammelbehälter (300) und der mindestens eine Kübel (82) komplementäre Strukturen umfassen, die so geformt sind, dass, wenn die komplementären Strukturen des ersten Sammelbehälters (300) und des mindestens einen Kübels (82) ineinander greifen, eine Bewegung des ersten Sammelbehälters (300) relativ zu dem mindestens einen Kübel (82) in allen Richtungen blockiert ist, außer entlang der vertikalen Richtung, nach oben und weg von dem mindestens einen Kübel (82), wobei insbesondere die komplementären Strukturen des ersten Sammelbehälters (300) und des mindestens einen Kübels (82) eine Aussparung (94) innerhalb der Aufnahme (89) des mindestens einen Kübels (82) und einen Umfang (323) mindestens eines Abschnitts des ersten Sammelbehälters (300) umfassen, wobei insbesondere die Aussparung (94) innerhalb der Aufnahme (89) des mindestens einen Kübels (82) auch mindestens einem Abschnitt eines Umfangs (323) des zweiten Sammelbehälters (300) entspricht, und optional
wobei einer von dem ersten Sammelbehälter (300) und dem zweiten Sammelbehälter (300) einen Deckel (460) umfasst, der bewegbar ist, um das jeweilige Sammelbehälter-Innenkompartiment (416) zu schließen und abzudichten, und der andere von dem ersten Sammelbehälter (300) und dem zweiten Sammelbehälter (300) oben offen ist, so dass das jeweilige Sammelbehälter-Innenkompartiment (416) offen und unversiegelt ist.

9. Abfragestation (10) nach Anspruch 5, ferner umfassend:
mindestens einen Probenbehälter (200) mit einem Körper, der einen Innenraum begrenzt, um eine biologische Probe zumindest teilweise zu umschließen, wobei der mindestens eine Probenbehälter (200) ferner einen drahtlosen Transponder umfasst, der physisch mit dem Körper verbunden ist, wobei der drahtlose Transponder (208) Identifikationsinformationen für die biologische Probe trägt,
wobei der Körper innerhalb der Vielzahl von Durchgangslöchern (326) des Sammelbehälters (300) aufnehmbar ist, so dass, wenn der Sammelbehälter (300) innerhalb der Aufnahme (89) des mindestens einen Kübels (82) aufgenommen ist und die Vielzahl von Durchgangslöchern (326) jeweils seitlich mit jeweiligen Antennen (34) der mindestens einen Anordnung (38) von Antennen (34) entlang der ersten und der zweiten seitlichen Richtung ausgerichtet sind, der drahtlose Transponder (208) seitlich mit einer der Antennen (34) der mindestens einen Anordnung (38) von Antennen (34) entlang der ersten und der zweiten seitlichen Richtung ausgerichtet ist.

10. Verfahren zum Übertragen biologischer Proben von einem Kryogenfrierer zu einem Probentransporter, wobei das Verfahren umfasst:
Befestigen eines ersten Kübels innerhalb eines Innenhohlraums einer Vertiefung (12) über einen Rahmen, so dass eine Bewegung des ersten Kübels relativ zum Rahmen und zur Vertiefung in einer oder mehreren Richtungen durch Ineinandergreifen komplementärer Strukturen des ersten Kübels und mindestens eines von Rahmen und Vertiefung blockiert ist;
Befestigen eines Sammelbehälters innerhalb einer Aufnahme des ersten Kübels, so dass eine Bewegung des Sammelbehälters relativ zum ersten Kübel in einer oder mehreren Richtungen blockiert ist;
Befestigen des Sammelbehälters relativ zu einer ersten Vielzahl von Antennen (34), die in einer ersten Ebene angeordnet sind, die unterhalb der Vertiefung positioniert ist, so dass eine Bewegung des Sammelbehälters relativ zur ersten Vielzahl von Antennen in einer oder mehreren Richtungen blockiert ist und jeweilige einer ersten Vielzahl von Durchgangslöchern (326a), die sich durch ein erstes Tablett des Sammelbehälters erstrecken, seitlich mit jeweiligen der ersten Vielzahl von Antennen entlang mindestens einer seitlichen Richtung ausgerichtet sind, wobei das erste Tablett innerhalb eines ersten Innenkompartiments positioniert ist, das durch einen Boden des Sammelbehälters und mindestens eine Seitenwand des Sammelbehälters begrenzt wird;
Befestigen des Probentransporters (400) relativ zu einer zweiten Vielzahl von Antennen, die in einer zweiten Ebene angeordnet sind, so dass eine Bewegung des Probentransporters relativ zu der zweiten Vielzahl von Antennen in einer oder mehreren Richtungen blockiert ist und jeweilige einer zweiten Vielzahl von Durchgangslöchern (326b), die sich durch ein zweites Tablett des Probentransporters erstrecken, seitlich mit jeweiligen der zweiten Vielzahl von Antennen entlang mindestens einer seitlichen Richtung ausgerichtet sind, wobei das zweite Tablett innerhalb eines zweiten Innenkompartiments positioniert ist, das durch einen Boden des Probentransporters und mindestens eine Seitenwand des Probentransporters begrenzt wird;
Befestigen der ersten Vielzahl von Antennen und der zweiten Vielzahl von Antennen an der Vertiefung mittels eines Kopplers und Anbringen des Kopplers an der Vertiefung an einer Position oberhalb des Bodens des Sammelbehälters, wenn der erste Kübel innerhalb des Innenhohlraums der Vertiefung befestigt ist und wenn der Sammelbehälter innerhalb der Aufnahme des ersten Kübels befestigt ist;
Entfernen eines Probenbehälters (200), der eine biologische Probe einschließt, aus einem der ersten Vielzahl von Durchgangslöchern, wodurch ein drahtloser Transponder (208), der physisch mit dem Probenbehälter verbunden ist, aus der Ausrichtung mit der jeweiligen der ersten Vielzahl von Antennen bewegt wird, die mit dem einen der ersten Vielzahl von Durchgangslöchern ausgerichtet ist;
Einsetzen des Probenbehälters (200) in eines der zweiten Vielzahl von Durchgangslöchern, wodurch der drahtlose Transponder, der physisch mit dem Probenbehälter verbunden ist, in Ausrichtung mit der jeweiligen der zweiten Vielzahl von Antennen bewegt wird, die mit dem einen der zweiten Vielzahl von Durchgangslöchern ausgerichtet ist;
nach dem Einsetzen des Probenbehälters in das eine der zweiten Vielzahl von Durchgangslöchern, Abdichten des zweiten Innenkompartiments, wodurch der Probenbehälter thermisch von einer Umgebung des Probentransporters isoliert wird; und
nach dem Einsetzen des Probenbehälters in das eine der zweiten Vielzahl von Durchgangslöchern, Bewegen des Probentransporters relativ zur zweiten Vielzahl von Antennen, wodurch alle der zweiten Vielzahl von Durchgangslöchern aus der Ausrichtung mit der zweiten Vielzahl von Antennen bewegt werden.

11. Verfahren nach Anspruch 10, ferner umfassend:
Befestigen eines zweiten Kübels innerhalb des Innenhohlraums der Vertiefung (12), so dass eine Bewegung des zweiten Kübels relativ zu der Vertiefung (12) in einer oder mehreren Richtungen durch Ineinandergreifen komplementärer Strukturen des zweiten Kübels und der Vertiefung blockiert ist;
wobei das Befestigen des Probentransporters relativ zu der zweiten Vielzahl von Antennen das Befestigen des Probentransporters innerhalb einer Aufnahme des zweiten Kübels umfasst, so dass eine Bewegung des Probentransporters relativ zu dem zweiten Kübel in einer oder mehreren Richtungen blockiert ist,
und wobei die zweite Vielzahl von Antennen unterhalb der Vertiefung positioniert ist.

12. Verfahren nach Anspruch 11, ferner umfassend:
Befüllen eines Teils der Aufnahme des ersten Kübels mit einem Kühlmittel bei oder unter -150 °C; und
Befüllen eines Teils der Aufnahme des zweiten Kübels mit einem Kühlmittel bei oder unter -150 °C.

13. Verfahren nach einem der Ansprüche 10 bis 12, ferner umfassend mindestens eines von:
a) vor dem Entfernen des Probenbehälters aus dem einen der ersten Vielzahl von Durchgangslöchern, Abfragen des drahtlosen Transponders mit der einen der ersten Vielzahl von Antennen, wodurch der Probenbehälter eindeutig von anderen Probenbehältern identifiziert wird, die innerhalb der ersten Vielzahl von Durchgangslöchern positioniert sind; und nach dem Einsetzen des Probenbehälters in das eine der zweiten Vielzahl von Durchgangslöchern, Abfragen des drahtlosen Transponders mit der einen der zweiten Vielzahl von Antennen, wodurch bestätigt wird, dass der Probenbehälter derselbe ist, der aus dem einen der ersten Vielzahl von Durchgangslöchern entfernt wurde,
b) vor dem Befestigen des Sammelbehälters relativ zu der ersten Vielzahl von Antennen, Entfernen des Sammelbehälters aus einer kryogenen Umgebung, die innerhalb des Kryogenfrierers eingeschlossen ist,
c) Halten der biologischen Probe bei oder unter -150 °C für die gesamte Dauer des Verfahrens,
d) Befestigen des Sammelbehälters relativ zu der ersten Vielzahl von Antennen umfassend das Positionieren des drahtlosen Transponders, der physisch mit dem Probenbehälter verbunden ist, innerhalb von 20 mm von der jeweiligen der ersten Vielzahl von Antennen, die mit dem einen der ersten Vielzahl von Durchgangslöchern ausgerichtet ist.

## Revendications

1. Station d'interrogation (10) comprenant:
un cadre (11) et un puits (12), ledit puits (12) présentant un fond (18), une face supérieure (22) opposée au fond (18) et au moins une paroi latérale (20) qui s'étend vers le haut à partir du fond (18) en direction de la face supérieure (22), dans laquelle le fond (18), ladite au moins une paroi latérale (20) et la face supérieure (22) délimitent ensemble une cavité intérieure (24) du puits (12), au moins une partie de la face supérieure (22) du puits (12) étant ouverte, le cadre (11) comprenant au moins une ouverture (26) qui fournit un passage à travers la face supérieure ouverte (22) du puits (12) de manière à fournir de l'accès à la cavité intérieure (24) depuis un extérieur de celle-ci;
au moins un réseau (38) d'antennes (34) disposées dans un plan (P1); et
un coupleur (56) qui fixe ledit au moins un réseau (38) d'antennes (34) sous le fond (18) du puits (12) à une distance perpendiculaire définie du fond (18) du puits (12) dans une direction verticale, et dans laquelle ledit au moins un réseau (38) d'antennes (34) est aligné latéralement sur une ouverture respective de ladite au moins une ouverture (26) du cadre (11);
la station d'interrogation étant **caractérisée par** au moins une carte de circuit imprimé (32) qui porte ledit au moins un réseau (38) d'antennes (34),
dans laquelle ledit coupleur (56) comprend une plaque fendue (60) qui supporte ladite au moins une carte de circuit imprimé (32) sur une position située entre le puits (12) et la plaque fendue (60), et la plaque fendue (60) comprend une pluralité de fentes qui s'étendent à travers une épaisseur de la plaque fendue (60) et qui sont alignées sur des composants montés selon la direction verticale sur une face inférieure (62) de ladite au moins une carte de circuit imprimé (32), et comprenant en option un matériau hydrophobe (74) positionné entre au moins une portion de la plaque fendue (60) et une portion de ladite au moins une carte de circuit imprimé (32), ladite au moins une portion de la plaque fendue (60) comprenant en particulier plusieurs portions discrètes de la plaque fendue (60) et/ou étant dépourvue de toutes fentes qui s'étendent à travers l'épaisseur de la plaque fendue (60).

2. Station d'interrogation (10) selon la revendication 1, dans laquelle
ladite au moins une ouverture (26) du cadre (11) comprend une première ouverture (26a) et
une deuxième ouverture (26b) qui sont séparées l'une de l'autre par une portion du cadre (11) qui est positionnée entre la première ouverture (26a) et la deuxième ouverture (26b) selon une direction latérale qui est perpendiculaire à la direction verticale;
ledit au moins un réseau (38) d'antennes (34), en option:
a) comprend un premier réseau d'antennes et un deuxième réseau d'antennes, et le premier réseau (38a) d'antennes (34) est aligné latéralement sur la première ouverture (26a) selon une première direction latérale et une deuxième direction latérale, et le deuxième réseau (38b) d'antennes (34) est aligné sur la deuxième ouverture (26b) selon la première direction latérale et la deuxième direction latérale, le premier réseau (38a) d'antennes (34) comprenant en particulier une pluralité de rangées d'antennes (34), chacune de la pluralité de rangées étant équidistante de rangées adjacentes parmi la pluralité de rangées selon la première direction latérale qui est perpendiculaire à la direction verticale, et une pluralité de colonnes d'antennes (34), chacune de la pluralité de colonnes étant équidistante de colonnes adjacentes parmi la pluralité de colonnes selon la deuxième direction latérale qui est perpendiculaire à la fois à la direction verticale et à la première direction latérale; et/ou
b) est positionné au-dessous du fond (18) du puits (12) sur une position où ledit au moins un réseau (38) d'antennes (34) se trouve à moins de 5 mm du fond (18) du puits (12), mesuré selon la direction verticale; et/ou
c) est positionné de telle sorte qu'un isolateur (54), en option compris par la station d'interrogation (10), soit positionné entre le fond (18) du puits (12) et ledit au moins un réseau (38) d'antennes (34).

3. Station d'interrogation (10) selon la revendication 1, dans laquelle le coupleur (56) comprend une paire de supports (76) qui fixent la plaque fendue (60) au puits (12), en particulier dans laquelle le coupleur (56) fixe la carte de circuit imprimé (32) au puits (12) sans enfermer entièrement la carte de circuit imprimé (32), et la station d'interrogation (10) comprend une unité intégrale dimensionnée pour être montée dans une ouverture au moins adjacente à une surface (114) d'une station de transfert (104).

4. Station d'interrogation (10) selon la revendication 1, comprenant en outre:
au moins un seau (82) comprenant un fond de seau (84) et au moins une paroi latérale de seau (88) s'étendant à partir du fond de seau (84) de sorte que le fond de seau (84) et ladite au moins une paroi latérale de seau (88) délimitent ensemble un réservoir (89) destiné à contenir un volume de fluide de refroidissement, dans laquelle ledit au moins un seau (82) peut être reçu dans ladite au moins une ouverture (26) du cadre (11) de sorte que le fond de seau (84) soit aligné latéralement sur ledit au moins un réseau (38) d'antennes (34) selon les première et deuxième directions latérales.

5. Station d'interrogation (10) selon la revendication 4, dans laquelle ledit au moins un seau (82) et le cadre (11) comprennent des structures complémentaires formées de telle sorte que, lorsque les structures complémentaires s'engagent les unes dans les autres, un mouvement dudit au moins un seau (82) par rapport au cadre (11) et au puits (12) soit bloqué dans toutes les directions, sauf vers le haut et dans la direction opposée au puits (12) selon la direction verticale, en particulier de telle sorte que
lorsque les structures complémentaires s'engagent les unes dans les autres et que le mouvement dudit au moins un seau (82) par rapport au cadre (11) et au puits (12) est bloqué dans toutes les directions, sauf vers le haut et dans la direction opposée au puits (12) selon la direction verticale: le fond de seau (84) est séparé du fond (18) du puits (12) par un espace d'air (112) et/ou une totalité dudit au moins un seau (82) est affleurante ou en retrait par rapport à une surface supérieure (106) du cadre (11).

6. Station d'interrogation (10) selon la revendication 5, dans laquelle les structures complémentaires comprennent un évidement (94) formé par le cadre (11) et au moins une portion d'un périmètre extérieur (323) dudit au moins un seau (82),
ledit au moins un seau (82) comprenant en particulier:
a) une poignée (92) qui est fixée de manière pivotante à ladite au moins une paroi latérale de seau (88), et l'évidement (94) comprend une portion destinée à recevoir la poignée (92), et/ou
b) un premier seau (82a) et un deuxième seau (82b), ledit premier seau (82a) étant identique audit deuxième seau (82b).

7. Station d'interrogation (10) selon l'une quelconque des revendications 4 à 6, comprenant en outre:
un réservoir collecteur (300) comprenant un fond de réservoir collecteur (304) et au moins une paroi latérale de réservoir collecteur (306) qui délimitent ensemble un compartiment intérieur de réservoir collecteur (316), le réservoir collecteur (300) comprenant un plateau (322) ayant une pluralité de trous traversants (326) s'étendant à travers celui-ci, chacun de la pluralité de trous traversants (326) étant dimensionné pour recevoir un conteneur d'échantillons (200) qui contient un échantillon biologique, dans laquelle ledit réservoir collecteur (300) peut être reçu à l'intérieur du réservoir (89) dudit au moins un seau (82) de sorte que la pluralité de trous traversants (326) soient alignés chacun latéralement sur des antennes (34) respectives dudit au moins un réseau (38) d'antennes (34), selon les première et deuxième directions latérales.

8. Station d'interrogation (10) selon la revendication 7, dans laquelle le réservoir collecteur (300) est un premier réservoir collecteur (300), la station d'interrogation (10) comprenant en outre un deuxième réservoir collecteur (300), ledit deuxième réservoir collecteur (300) comprenant un deuxième plateau (322) ayant une deuxième pluralité de trous traversants (326b) s'étendant à travers celui-ci, chacun de la deuxième pluralité de trous traversants (326b) étant dimensionné pour recevoir un conteneur d'échantillons (200) qui contient un échantillon biologique, dans laquelle ledit deuxième réservoir collecteur (300) peut être reçu à l'intérieur du réservoir (89) dudit au moins un seau (82) de sorte que la deuxième pluralité de trous traversants (326b) soient alignés chacun latéralement sur des antennes (34) respectives dudit au moins un réseau (38) d'antennes (34), selon les première et deuxième directions latérales,
ledit premier réservoir collecteur (300) pouvant en particulier être reçu dans l'un dudit au moins un seau (82) de sorte que la pluralité de trous traversants (326) du premier réservoir collecteur (300) soient alignés chacun latéralement sur un premier sous-ensemble dudit au moins un réseau (38) d'antennes (34) selon les première et deuxième directions latérales, et le deuxième réservoir collecteur (300) peut être simultanément reçu à l'intérieur d'un autre dudit au moins un seau (82) de sorte que la deuxième pluralité de trous traversants (326b) soient chacun alignés latéralement sur un deuxième sous-ensemble dudit au moins un réseau (38) d'antennes (34) selon les première et deuxième directions latérales, et aucune des antennes (34) du premier sous-ensemble n'est incluse dans le deuxième sous-ensemble, et, en option,
dans lequel le premier réservoir collecteur (300) et ledit au moins un seau (82) comprennent des structures complémentaires formées de telle sorte que, lorsque les structures complémentaires du premier réservoir collecteur (300) et dudit au moins un seau (82) s'engagent les unes dans les autres, un mouvement du premier réservoir collecteur (300) par rapport audit au moins un seau (82) soit bloqué dans toutes les directions, sauf selon la direction verticale, vers le haut et dans la direction opposée audit au moins un seau (82), en particulier, dans laquelle lesdites structures complémentaires du premier réservoir collecteur (300) et dudit au moins un seau (82) comprennent un évidement (94) dans le réservoir (89) dudit au moins un seau (82) et un périmètre (323) d'au moins une portion du premier réservoir collecteur (300), en particulier dans laquelle l'évidement (94) dans le réservoir (89) dudit au moins un seau (82) correspond également à au moins une portion d'un périmètre (323) du deuxième réservoir collecteur (300), et, en option,
dans laquelle un parmi le premier réservoir collecteur (300) et le deuxième réservoir collecteur (300) comprend un couvercle (460) qui est mobile pour fermer et sceller le compartiment intérieur de réservoir collecteur (416) respectif, et l'autre parmi le premier réservoir collecteur (300) et le deuxième réservoir collecteur (300) est ouverte sur le dessus de sorte que le compartiment intérieur de réservoir collecteur (416) respectif est ouvert et non scellé.

9. Station d'interrogation (10) selon la revendication 5, comprenant en outre:
au moins un conteneur d'échantillons (200) avec un corps qui délimite un intérieur afin d'entourer, au moins en partie, un échantillon biologique, ledit au moins un conteneur d'échantillons (200) comprenant en outre un transpondeur sans fil qui est physiquement associé au corps, ledit transpondeur sans fil (208) portant des informations d'identification pour l'échantillon biologique,
dans laquelle ledit corps peut être reçu dans la pluralité de trous traversants (326) du réservoir collecteur (300) de sorte que, lorsque le réservoir collecteur (300) est reçu dans le réservoir (89) dudit au moins un seau (82) et que la pluralité de trous traversants (326) sont alignés chacun latéralement sur des antennes (34) respectives dudit au moins un réseau (38) d'antennes (34) selon les première et deuxième directions latérales, le transpondeur sans fil (208) est aligné latéralement sur l'une des antennes (34) dudit au moins un réseau (38) d'antennes (34), selon les première et deuxième directions latérales.

10. Procédé destiné à transférer des échantillons biologiques depuis un congélateur cryogénique vers un transporteur d'échantillons, le procédé comprenant:
fixer un premier seau à l'intérieur d'une cavité intérieure d'un puits (12) au moyen d'un cadre de sorte qu'un mouvement du premier seau par rapport au cadre et au puits soit bloqué dans une ou plusieurs directions par l'engagement de structures complémentaires du premier seau et d'au moins un parmi le cadre et le puits;
fixer un réservoir collecteur dans un réservoir du premier seau de sorte qu'un mouvement du réservoir collecteur par rapport au premier seau soit bloqué dans une ou plusieurs directions;
fixer le réservoir collecteur par rapport à une première pluralité d'antennes (34) disposées dans un premier plan qui est positionné au-dessous du puits, de manière à bloquer un mouvement du réservoir collecteur par rapport à la première pluralité d'antennes dans une ou plusieurs directions et à aligner latéralement des trous traversants respectifs d'une première pluralité de trous traversants (326a) s'étendant à travers un premier plateau du réservoir collecteur, sur des antennes respectives de la première pluralité d'antennes, au moins selon une direction latérale, dans lequel le premier plateau est positionné à l'intérieur d'un premier compartiment intérieur délimité par un fond du réservoir collecteur et au moins une paroi latérale du réservoir collecteur;
fixer le transporteur d'échantillons (400) par rapport à une deuxième pluralité d'antennes disposées dans un deuxième plan, de manière à bloquer un mouvement du transporteur d'échantillons par rapport à la deuxième pluralité d'antennes dans une ou plusieurs directions et à aligner latéralement des trous traversants respectifs d'une deuxième pluralité de trous traversants (326b) s'étendant à travers un deuxième plateau du transporteur d'échantillons, sur des antennes respectives de la deuxième pluralité d'antennes, selon au moins une direction latérale, dans lequel le deuxième plateau est positionné dans un deuxième compartiment intérieur délimité par un fond du transporteur d'échantillons et au moins une paroi latérale du transporteur d'échantillons;
fixer la première pluralité d'antennes et la deuxième pluralité d'antennes au puits à l'aide d'un coupleur, et attacher le coupleur au puits sur une position située au-dessus du fond du réservoir collecteur lorsque le premier seau est fixé dans la cavité intérieure du puits et lorsque le réservoir collecteur est fixé dans le réservoir du premier seau;
retirer un conteneur d'échantillons (200) renfermant un échantillon biologique de l'un de la première pluralité de trous traversants, déplaçant ainsi un transpondeur sans fil (208) physiquement associé au conteneur d'échantillons, hors de l'alignement avec l'antenne respective de la première pluralité d'antennes, qui est alignée sur ledit un de la première pluralité de trous traversants;
insérer le conteneur d'échantillons (200) dans l'un de la deuxième pluralité de trous traversants, déplaçant ainsi le transpondeur sans fil physiquement associé au conteneur d'échantillons, en alignement avec l'antenne respective de la deuxième pluralité d'antennes, qui est alignée sur ledit un de la deuxième pluralité de trous traversants;
après avoir inséré le conteneur d'échantillons dans ledit un de la deuxième pluralité de trous traversants, sceller le deuxième compartiment intérieur, isolant ainsi thermiquement le conteneur d'échantillons d'un environnement extérieur du transporteur d'échantillons; et
après avoir inséré le conteneur d'échantillons dans ledit un de la deuxième pluralité de trous traversants, déplacer le transporteur d'échantillon par rapport à la deuxième pluralité d'antennes, déplaçant ainsi l'ensemble de la deuxième pluralité de trous traversants hors de l'alignement avec la deuxième pluralité d'antennes.

11. Procédé selon la revendication 10, comprenant en outre:
fixer un deuxième seau dans la cavité intérieure du puits (12) de sorte qu'un mouvement du deuxième seau par rapport au puits (12) soit bloqué dans une ou plusieurs directions par l'engagement de structures complémentaires du deuxième seau et du puits;
dans lequel la fixation du transporteur d'échantillon par rapport à la deuxième pluralité d'antennes comprend la fixation du transporteur d'échantillon dans un réservoir du deuxième seau de manière à bloquer un mouvement du transporteur d'échantillon par rapport au deuxième seau dans une ou plusieurs directions,
et dans lequel la deuxième pluralité d'antennes est positionnée au-dessous du puits.

12. Procédé selon la revendication 11, comprenant en outre:
remplir une partie du réservoir du premier seau d'un fluide de refroidissement à une température inférieure ou égale à -150 °C; et -
remplir une partie du réservoir du deuxième seau d'un fluide de refroidissement à une température inférieure ou égale à -150 °C.-

13. Procédé selon l'une quelconque des revendications 10 à 12, comprenant en outre au moins une des étapes suivantes:
a) avant de retirer le conteneur d'échantillons dudit un de la première pluralité de trous traversants, interroger le transpondeur sans fil avec ladite une de la première pluralité d'antennes, identifiant ainsi de manière unique le conteneur d'échantillons parmi d'autres conteneurs d'échantillons qui sont positionnés à l'intérieur de la première pluralité de trous traversants; et après avoir inséré le conteneur d'échantillons dans ledit un de la deuxième pluralité de trous traversants, interroger le transpondeur sans fil avec ladite une de la deuxième pluralité d'antennes, confirmant ainsi que le conteneur d'échantillons est le même que celui qui a été retiré dudit un de la première pluralité de trous traversants;
b) avant de fixer le réservoir collecteur par rapport à la première pluralité d'antennes, retirer le réservoir collecteur d'un environnement cryogénique enfermé à l'intérieur du congélateur cryogénique,
c) maintenir l'échantillon biologique à une température inférieure ou égale à - 150 °C pendant toute la durée du procédé,-
d) fixer le réservoir collecteur par rapport à la première pluralité d'antennes, comprenant le positionnement du transpondeur sans fil associé physiquement au conteneur d'échantillons, à l'intérieur de 20 mm de l'antenne respective de la première pluralité d'antennes, qui est alignée sur ledit un de la première pluralité de trous traversants.
